# EUROPÄISCHE PATENTSCHRIFT

(11) **EP 1 034 309 B1**
(45) Veröffentlichungstag und Bekanntmachung des Hinweises auf die Patenterteilung: **08.05.2002**
(21) Anmeldenummer: 98966504.7
(22) Anmeldetag: 27.11.1998
(51) Int. Cl.: C12Q 1/68

(54) **VERFAHREN ZUR HERSTELLUNG KOMPLEXER DNA-METHYLIERUNGS-FINGERABDRÜCKE**
METHOD FOR PRODUCING COMPLEX DNA METHYLATION FINGERPRINTS
PROCEDE DE PRODUCTION D'EMPREINTES DIGITALES COMPLEXES A METHYLATION D'ADN

(30) Priorität: 27.11.1997 DE 19754482
(43) Veröffentlichungstag der Anmeldung: 13.09.2000
(73) Patentinhaber: Epigenomics AG, 10435 Berlin (DE)
(72) Erfinder: OLEK, Alexander, D-10781 Berlin (DE); OLEK, Sven, Stefan, D-69117 Heidelberg (DE); WALTER, Jörn, D-14057 Berlin (DE)
(74) Vertreter: Schubert, Klemens, Dr.
(86) Internationale Anmeldenummer: DE9803558
(87) Internationale Veröffentlichungsnummer: WO9928498

(56) Entgegenhaltungen:
- WO-A-95/15373
- WO-A-97/45560
- WO-A-97/46705
- XIONG Z AND LAIRD P W: "COBRA: a sensitive and quantitative DNA methylation assay" NUCLEIC ACIDS RESEARCH, Bd. 25, Nr. 12, 1997, Seiten 2532-2534, XP002106407 in der Anmeldung erwähnt
- OLEK A ET AL.: "A modified an improved method for bisulphite based cytosine methylation analysis " NUCLEIC ACIDS RESEARCH, Bd. 24, Nr. 24, 1996, Seiten 5064-5066, XP002106408
- GONZALGO M L AND JONES P A: "Rapid quantification of methylation differences at specific sites using methylation-sensitive single nucleotide primer extension (Ms-SNuPE)" NUCLEIC ACIDS RESEARCH, Bd. 25, Nr. 12, 1997, Seiten 2529-2531, XP002106409 in der Anmeldung erwähnt
- POGRIBNY I P AND JAMES S J: "A method to estimate the percent loss of cytosine methyl groups at defined CpG sites in liver DNA from methyl-deficient rats" NUTRITIONAL BIOCHEMISTRY, Bd. 8, 1997, Seiten 355-359, XP002106410
- GRIGG G AND CLARK S: "Sequencing 5-methylcytosine residues in genomic DNA" BIOESSAYS, Bd. 16, Nr. 6, 1994, Seiten 431-436, XP002106411
- ZESCHNIGH M ET AL.: "Imprinted segments in the human genome: different DNA methylation patterns in the Prader-Willi/Angelman syndrome region as determined by the genomic sequencing method" HUMAN MOLECULAR GENETICS, Bd. 6, Nr. 3, 1997, Seiten 387-395, XP002106412
- FEIL R ET AL.: "Methylation analysis on individual chromosomes: improved protocol for bisulphite genomic sequencing" NUCLEIC ACIDS RESEARCH, Bd. 22, Nr. 4, 1994, Seiten 695-696, XP002106413
- MARTIN V ET AL: "Genomic sequencing indicates a correlation between DNA hypomethylation in the 5' region of the pS2 gene and its expression in human breast cancer cell lines" GENE, Bd. 157, Nr. 1, 19. Mai 1995, Seite 261-264 XP004042334
- EL-MAARRI, O ET AL.: "Methylation levels at selected CpG sites in the factor VIII and FGFR3 genes, in mature female and male germ cells: Implications for male-driven evolution" AMERICAN JOURNAL OF HUMAN GENETICS, Bd. 63, 1998, Seiten 1001-1008, XP002106414

## Beschreibung

### 1. Gebiet der Erfindung

Das hier zu patentierende Verfahren schafft eine neue Möglichkeit zur differentiellen Diagnose von Krebserkrankungen. Es führt zu einem vertieften Verständnis der Carcinogenese und der Pathogenese der polygen vererbten Krankheiten. Das Verfahren verspricht weiterhin die Identifizierung von an der Entstehung von Krankheiten beteiligten Genen. Nach wie vor ist die Zelldifferenzierung und die Differenzierung eines höheren Organismus im wesentlichen nicht verstanden. Auch hier verspricht das Verfahren erheblichen Erkenntnisgewinn.

Die nach den methodischen Entwicklungen der letzten Jahre in der Molekularbiologie gut studierten Beobachtungsebenen sind die Gene selbst, die Übersetzung dieser Gene in RNA und die daraus entstehenden Proteine. Wann im Laufe der Entwicklung eines Individuums welches Gen angeschaltet wird und wie Aktivieren und Inhibieren bestimmter Gene in bestimmten Zellen und Geweben gesteuert wird, ist mit hoher Wahrscheinlichkeit mit Ausmaß und Charakter der Methylierung der Gene bzw. des Genoms korrelierbar. Insofern ist die Annahme naheliegend, daß pathogene Zustände sich in einem veränderten Methylierungsmuster einzelner Gene oder des Genoms äußern.

Stand der Technik ist ein Verfahren, welches das Studium von Methylierungsmustern einzelner Gene gestattet. Jüngere Fortentwicklungen dieser Methode erlauben auch die Analyse kleinster Mengen Ausgangsmaterial, wobei sich aber die Gesamtzahl der Meßpunkte immer noch höchstens im zweistelligen Bereich von theoretisch mindestens 10⁷ Meßpunkten befindet. Mit Hilfe des zu patentierenden Verfahrens können nun erstmalig beliebige Ausschnitte des Genoms mit beliebig vielen Meßpunkten untersucht werden. Damit ermöglicht das Verfahren die Identifizierung von auf andere Art nicht zu ermittelnden Ursachen für genetische Krankheiten aller Art und ermöglicht die Entwicklung neuer Behandlungsstrategien und Identifizierung von Ziel-Proteinen für neue Medikamente.

### 2. Stand der Technik

### 2.1. Stand der Technik der molekularen Analyse von Zell-Phänotypen

Studium der Genexpression kann auf der Ebene der RNA und auf der Ebene der Proteine stattfinden. Beide Ebenen reflektieren im Prinzip wichtige phänotypische Parameter. Proteinuntersuchungen mit Hilfe zweidimensionaler Gele (McFarrel-Verfahren) sind seit ca. 15 Jahren bekannt. Man kann damit in einer Analyse die chromatographische Position von einigen Tausend Proteinen darstellen. Schon sehr früh wurden solche Elektropherogramme auch mit Mitteln der EDV bearbeitet bzw. ausgewertet. Die Aussagekraft der Methode ist im Prinzip hoch, sie ist aber den modernen Methoden der auf der RNA-Analyse beruhenden Genexpression in zwei Punkten unterlegen.

Die Detektion vor allem von regulatorisch wichtigen Proteinen aus geringen Mengen von Zellen scheitert aber an der viel zu geringen Sensitivität der eingesetzten Methoden. Proteine können im Gegensatz zu Nukleinsäuren nämlich nicht amplifiziert werden. Außerdem handelt es sich um hoch-komplexe, nicht zu automatisierende und sehr teure Verfahren. Die Analyse der RNA hat dem gegenüber erhebliche Vorteile. Sie ist wegen des Einsatzes der PCR empfindlicher. Vor allem aber kann man jede RNA-Spezies, die als interessant erkannt worden ist, sofort in seiner Sequenz identifiziert werden.

Über- oder Unterexpression einzelner RNA's mit bekannter Sequenz sind meist leicht nachzuweisen, sind aber nur in Ausnahmefällen im Zusammenhang mit den hier zur Debatte stehenden Anwendungen aussagekräftig.

Die Methode des sogenannten "diffential displays" gestattet ein bestenfalls halbquantitatives Studium der Expression. Per PCR amplifizierte Expressionsprodukte werden in der Gelektrophorese aufgetrennt. Begrenzt wird die Aussagekraft durch das Auflösungsvermögen der Gelektrophorese. Die Methode ist außerdem bei weitem nicht sensitiv und robust genug für die Anwendung in der Routinediagnostik (Liang, P. and Pardee, A.B., Science 257, 967-971).

Stark über- oder unterexprimierte Gene werden häufig durch subtraktive Techniken identifiziert. Dabei werden cDNA-Klone einer zu untersuchenden Zell- oder Gewebespezies ausplattiert. Dagegen wird cDNA aus Vergleichsmaterial hybridisiert. Expressionsmuster kann man damit nicht zuverlässig erstellen.

Eine Aktivität des amerikanischen "Human Genome Project" ist das systematische Sequenzieren von exprimierten Genen. Die daraus sich ergebenden Informationen können genutzt werden, um Expressions-Chips zu bauen, die das Studium praktisch aller exprimierten Sequenzen einer Zelloder Gewebeart in einem einzigen Experiment gestatten.

### 2.2. Stand der Technik bei der Analyse von Krebserkrankungen

Auslöser für eine Krebserkrankung, also das Entarten einer Zelle, sind immer Mutationen in Genen. Verursacher für diese Mutationen können exogene Einflüsse, aber auch Ereignisse in der Zelle sein. In wenigen Ausnahmefällen läßt eine einzige Mutation, die dann allerdings häufig größere Bereiche des Genoms trifft (Translokationen, Deletionen) die Zelle entarten, meist handelt es sich wohl um eine Kette von Mutationen auf verschiedenen Genen, die erst zusammen die bösartige Erkrankung bewirken. Diese Ereignisse auf DNA-Ebene spiegeln sich auf RNA- und Proteinebene wieder. Dabei tritt mit hoher Wahrscheinlichkeit eine Vervielfältigung ein, weil sicherlich in vielen Fällen Menge und Art der einen RNA das Ausmaß der Synthese einiger anderer RNA-Spezies beeinflußt. Dies führt zu veränderten Syntheseraten der entsprechenden Proteine, was seinerseits den Stoffwechsel entgleisen lassen kann und somit Mechanismen der Regulation und Gegenregulation in Gang setzt. Das Ergebnis ist ein auf ganz spezifische (aber weitgehend unbestimmbare) Art und Weise verändertes Genexpressionsmuster der betroffenen Zellen -spezifisch für ein bestimmtes Karzinom und spezifisch für das Stadium des Karzinoms und spezifisch für den Grad der Bösartigkeit des Karzinoms. Solche Phänomene entzogen sich bis heute jeder naturwissenschaftlichen Betrachtung. Es gab nämlich keine Möglichkeit, die Genexpression oder den Stoffwechsel einer Zelle in seiner Gesamtheit zu untersuchen. Mit der Chip-Technologie hat sich zum ersten Male eine solche Möglichkeit ergeben (Schena, M. et al., Science 270,467-470).

Will man das diagnostische Problem Frühdiagnostik von Tumoren auf molekularer Ebene lösen, wird man zum gegenwärtigen Zeitpunkt - von wenigen Ausnahmen abgesehen - mit einer unüberbrückbaren Schwierigkeit konfrontiert: Da man bei den meisten Tumoren nur bruchstückhaft die ursächlichen molekularen Ereignisse, also die verschiedenen Mutationen überblickt, weiß man beim medizinischen Untersuchungsmaterial nicht, wonach man suchen soll. Das heißt, man kann sich die ungeheure Empfindlichkeit und Spezifität der Polymerase-Kettenreaktion gar nicht zunutze machen. Ausnahmen sind z.B. bestimmte Darmtumore, das Ewing-Sarkom und bestimmte Leukämieformen, die tatsächlich jeweils durch eine einzige genau umschriebene Mutation definiert werden. Hier kann man die eine entartete Zelle unter Millionen normalen Zellen identifizieren. Allerdings gibt es auch innerhalb dieser scheinbar eindeutig definierten Tumorgruppen dermaßen unterschiedliche Verhaltensweisen, daß gefolgert werden muß, daß weitere unbekannte genetische Parameter (wie zum Beispiel auch der genetische Hintergrund des Individuums) eine wichtige Rolle spielen. Die immunologischen Tumormarker sind Hilfsgrößen, die nach wie vor nur einen bescheidenen Beitrag neben den übrigen konventionellen Diagnoseparametern darstellen. Diese können allerdings dazu dienen, verdächtige Zellen vorzuselektieren.

Eine unverzichtbar wichtige Rolle spielt die Histologie bei der Identifizierung entarteten Gewebes, aber eben nicht in der Früherkennung.

Da also auf molekularer Ebene die meisten Tumore für diagnostische Zwecke nicht hinreichend charakterisiert sind, gibt es auch in aller Regel keine Möglichkeit, eine Stadieneinteilung oder gar Einteilung nach Gefährlichkeitsgraden vorzunehmen. Eine solche Einteilung ist aber unabdingbar für eine verbesserte Einstellung der Behandlungen und vor allem auch für die Entwicklung effektiver neuer Medikamente und der Gentherapie.

### 2.3. Stand der Technik in der Erforschung der Zahl, Art und Eigenschaften der möglichen stabilen Zustände von Zellen höherer Organismen

In jüngerer Zeit mehren sich Hinweise, daß komplexe Regelsysteme (für welche die Steuerung von Zellen ein herausragendes Beispiel sind) -, sich selbst überlassen, oberhalb einer kritischen Minimalkomplexität und unterhalb einer kritischen Maximal-Konhektivität (der durchschnittlichen Zahl der Komponenten, mit denen eine beliebige Komponente verknüpft ist) - nur in einer begrenzten Zahl stabiler Zustände existieren können. (Kauffman, S.A., Origins of Order, Oxford University Presse, 1993). Dabei ist das Wort Zustand in diesem Zusammenhang als der Begriff der Wahl für das allgemeine Phänomen zu verstehen. Im Zusammenhang mit Zellen als biologischen Regelsystemen kann auch vom Differenzierungszustand oder Zelltyp gesprochen werden. Obwohl kein solcher Zusammenhang oder auch nur die Begrenzung der möglichen Zustände für biologische Systeme nachgewiesen wurde, wären die praktischen Implikationen von unabsehbarer Wichtigkeit: Gäbe es bei konstantem Informationsgehalt der Zellen eines Organismus (de facto ist dies innerhalb einer Spezies weitgehend der Fall) nur eine limitierte Anzahl von stabilen Zuständen, so wäre es wahrscheinlich, daß sich auch entartete Zellen nur in einem dieser Zustände oder im Übergang zwischen den möglichen Zuständen befinden können. Zur Zeit gibt es keine Möglichkeit, diese Zustände molekular zu definieren. Eine Korrelation zwischen den einzelnen Zuständen und dem Verhalten von Zellen ist nach dem Stand der Technik kaum zu verwirklichen. Eine solche Analyse könnte aber einen entscheidenden Beitrag zur Diagnostik und Prognostik von Erkrankungen leisten. Möglicherweise wäre sogar eine Korrelation der möglichen Zustände von erkrankten Zellen und der am besten ansprechenden Therapie möglich. Es ist weiterhin wahrscheinlich, daß eine solche Methode auch die Wahl des Zeitpunktes einer Behandlung entscheidend beeinflussen könnte. Fände man zum Beispiel, daß sich die Zellen eines Tumors auf einem Übergang zwischen möglichen Zuständen befinden, so kann angenommen werden, daß eine solche Population von Zellen einem durch die Behandlung entstehenden Selektionsdruck nachgiebiger wäre und somit leichter entkommen könnte. Eine Zellpopulation hat in einem solchen Szenario innerhalb solcher Übergangszustände eine wesentlich erhöhte Flexibilität und würde leicht in einen möglichen stabilen Zustand gedrängt werden, in dem der Selektionsdruck wegfiele, die Behandlung also effektlos wäre. Ein Verfahren, welches Zellen und Zellgruppen nach Zuständen klassifizieren könnte, würde also dazu beitragen, solche Probleme zu erkennen, zu verstehen und gegebenenfalls zu lösen. Nach dem Stand der Technik ist es nicht möglich, zu bestimmen ob es nur eine limitierte Anzahl von Zuständen von Zellen gibt. Daraus folgt, daß es nicht möglich ist, Gruppen von Zellen nach einem abstrakten Kriterium ihren Zuständen nach zu differenzieren und diese Zustände mit einem bestimmten Verhalten der Zellen vorherzusagen.

### 2.4. Erbliche Erkrankungen

Heute besteht die genetische Karte des menschlichen Genoms aus 2.500 sogenannten Mikrosatelliten. Dieses Instrumentarium nimmt man in Anspruch, um eine Vielzahl von Genen, meist solche, die im Defektfall eine genetische Erkrankung verursachen, per Kopplungsanalyse zu lokalisieren und schließlich zu identifizieren. Die häufigen, schwerwiegenden und durch ein einziges Defektgen verursachten genetischen Krankheiten sind so aus der Sicht des Genetikers aufgeklärt. Im Prinzip sollten auch die polygenen Krankheiten auf diese Art und Weise verstanden werden können. Viele polygene Krankheiten sind sehr häufig, so häufig, daß sie zu den sogenannten Volkskrankheiten gezählt werden. Asthma und Diabetes sind solche Beispiele. Viele Karzinomarten zählen ebenfalls dazu. Die Anwendung der oben geschilderten Strategie der Kopplungsanalyse brachte auch enorme Anfangserfolge. Es sind zahlreiche Verursachergene von wichtigen polygenen Krankheiten wie Diabetes, Schizophrenie, Atheriosklerose und Fettsucht gefunden worden. Neben den eigentlichen molekularbiologischen Labortechniken ist die entscheidende Voraussetzung zur genetischen Aufklärung dieser Krankheiten die Verfügbarkeit einer relativ großen Zahl der von der jeweiligen Krankheit betroffenen Patienten und Verwandten. Man mußte in den letzten zwei Jahren feststellen, daß die ursprünglich angenommene Anzahl von einigen hundert Patienten für die Kopplungsanalyse von polygenen Erkrankungen mit hoher Wahrscheinlichkeit eine Größenordnung zu tief liegt. Das gilt auf jeden Fall dann, wenn es sich um komplette Aufklärung des Spektrums der Verursachergene handeln soll. Da das Ausmaß der praktischen Arbeit, die für solch eine Kopplungsanalyse geleistet wird aber außerordentlich hoch ist, kann man nur noch mit ganz langsamen Fortschritten bei der Analyse polygener Krankheiten rechnen. Da gerade diese Krankheiten von einer ganz enormen sozialen und wirtschaftlichen Bedeutung sind, wird nach alternativen Strategien gesucht.

### 2.5. Stand der Technik DNA-Chips

Von allen Entwicklungen am weitesten fortgeschritten ist zweifellos das Prinzip von Affimetrix (z.B. U.S.-Patente 5,593,839, 5,999,695 oder 5,631,734). Aber auch eine Anzahl anderer Firmen und Forschungsprojekte haben DNA-Chips mit verschiedenen Eigenschaften für spezielle Anwendungen produziert (z.B. U.S.-Patente 5,667,667, 5,525,464 oder 5,492,806 oder z.B. Goffeau, A., Nature 385, 202-203; Weiler, J. and Hoheisel, J., Anal. Biochem. 243,218-227; Chee, M. et al., Science 274, 610-614). Die jüngsten Publikationen berichten schon über einen kommerziell verfügbaren HIV-Chip, der die Untersuchung des kompletten HIV-Genoms gestattet. Gegen bis zu 400.000 Oligonukleotide werden fluoreszenzmarkierte PCR-Produkte der zu untersuchenden Probe hybridisiert. Die Auswertung der Signale erfolgt mit Hilfe von CCD-Kameras. Es wird die seit langem bekannte Fähigkeit solcher Systeme zur allelspezifischen Hybridisierung genutzt. Das bedeutet: Nur dort, wo die Probe absolut komplementär zu einem fixierten Oligonukleotid ist, bleibt am Ende der Hybridisierungs- und Waschprozeduren ein Signal erhalten. Die Untersuchung einer bekannten Gensequenz auf Mutationen gelingt, weil sich nicht nur jeder Teilbereich der gesamten Sequenz in Form von Oligonukleotidsequenzen auf der Matrix befindet, sondern weil dies auch für jede mögliche Abweichung von der Normalsequenz zutrifft. Die Effizienz der Chip-Prozedur rührt zu einem Teil daher, daß mit zwei einfachen Arbeitsschritten, nämlich der Hybridisierung und dem Waschen, die Sequenzinformation einer Vielzahl von Genen oder Genorten erhalten wird.

### 2.6. Analysemethoden zur Längenmessung

Einige Ausführungsvarianten des erfindungsgemäßen Verfahrens erfordern am Ende der Prozedur eine extrem schnelle und genaue Massebestimmung. Da für jeden von Zehntausenden Datenpunkten eine Fragment-Längenmessung durchgeführt werden muß, ist ein extrem effizientes Meßsystem erforderlich. Dafür kommen nach dem Stand der Technik automatische Sequenziergeräte (U.S.-Patent 4,811,218), Kapillarelektrophorese (z.B. Woolley. A.T., et al., Anal. Chem. 68, 4081-4086), MALDI-TOF (Siegert, C.W., et al. Anal. Biochem. 253, 55-65) und Auftrennungen mittels chemischer Markierungen (WO 95/04160) in Frage. Der Stand der Technik erlaubt es, wenn auch durch erhebliche Modifikationen und Einbindung in die neuartige Logik des erfindungsgemäßen Verfahrens, dieses effizient durchzuführen.

### 2.6.1. Massenspektrometrische Verfahren

Kurze DNA-Sequenzen können in MALDI-TOF Massenspektrometern präzise auf ihre Masse untersucht werden. Weiterhin gibt es nach dem Stand der Technik Verfahren, welche diese Analysenmethode mit Primer-Extension Reaktionen kombinieren. Dabei wird zum Beispiel ein Oligonukleotid spezifischer Sequenz mit einer DNA-Probe hybridisiert und pro Reaktion nur eines der vier Nukleotide hinzugegeben. Welches der Nukleotide nach der Hybridisierung von einer Polymerase an das 3'-Ende des Oligonukleotids angebracht wird, erlaubt die Bestimmung der Identität der Base hinter dem 3'-Terminus des Oligonukleotids. Es gibt auch unter anderem eine Variante dieser Methode, welche die Bestimmung der Länge von solchen repititiven Sequenzen erlaubt, die nur zwei der vier möglichen Basen enthält. Dabei werden die zu den vorkommenden Basen komplementären natürlichen Nukleotide und ein oder beide weiteren, derart modifizierten Nukleotide als Terminatoren zur Polymerase-Reaktion hinzugegeben, daß hinter der repititiven Sequenz die Reaktion zum erliegen kommt. Normalerweise sind die Terminatoren ddNTPs. Aus der Längenmessung kann dann die Länge der repititiven Sequenz abgeleitet werden.

### 2.7. Stand der Technik Methylierungsanalyse

Die Modifikation der genomischen Base Cytosin zu 5'-Methylcytosin stellt den bis heute wichtigsten und bestuntersuchten epigenetischen Parameter dar. Dennoch gibt es bis heute zwar Methoden, umfassende Genotypen von Zellen und Individuen zu ermitteln, aber noch keine vergleichbaren Ansätze auch in großem Maße epigenotypische Information zu generieren und auszuwerten.

Es gibt im Prinzip drei prinzipiell verschiedene Methoden den 5-Methyl-Status eines Cytosins im Sequenzkontext zu bestimmen.

Die erste prinzipielle Methode beruht auf der Verwendung von Restriktionsendonukleasen (RE), welche "methylierungssensitiv" sind. REs zeichnen sich dadurch aus, daß sie an einer bestimmten DNA-Sequenz, meist zwischen 4 und 8 Basen lang, einen Schnitt in die DNA einführen. Die Position solcher Schnitte kann dann durch Gelelektrophorese, Transfer auf eine Membran und Hybridisierung nachgewiesen werden. Methylierungssensitiv bedeutet, daß bestimmte Basen innerhalb der Erkennungssequenz unmethyliert vorliegen müssen, damit der Schnitt erfolgen kann. Das Bandenmuster nach einem Restriktionsschnitt und Gelektrophorese ändert sich also je nach Methylierungsmuster der DNA. Allerdings befinden sich die meisten methylierbaren CpG außerhalb von Erkennungssequenzen von REs, können also nicht untersucht werden.

Die Empfindlichkeit dieser Methoden ist extrem niedrig (Bird, A.P., Southern, E.M., J.Mol. Biol. 118, 27-47). Eine Variante kombiniert PCR mit dieser Methode; eine Amplifikation durch zwei auf beiden Seiten der Erkennungssequenz liegende Primer erfolgt nach einem Schnitt nur dann, wenn die Erkennungssequenz methyliert vorliegt. Die Empfindlichkeit steigt in diesem Fall auf theoretisch ein einziges Molekül der Zielsequenz, allerdings können mit hohem Aufwand nur einzelne Positionen untersucht werden (Shemer, R. et al., PNAS 93, 6371-6376).

Die zweite Variante beruht auf partieller chemischer Spaltung von Gesamt-DNA, nach dem Vorbild einer Maxam-Gilbert Sequenzierreaktion, Ligation von Adaptoren an die so generierten Enden, Amplifikation mit generischen Primern und Auftrennung auf einer Gelektrophorese. Mit diesem Verfahren können definierte Bereiche bis zur Größe von weniger als tausend Basenpaaren untersucht werden. Das Verfahren ist allerdings so kompliziert und unzuverlässig, daß es praktisch nicht mehr verwendet wird (Ward, C. et al., J. Biol. Chem. 265, 3030-3033).

Eine neue Methode zur Untersuchung von DNA auf 5-Methylcytosin beruht auf der spezifischen Reaktion von Bisulphit mit Cytosin. Dieses wird unter den entsprechenden Bedingungen in Uracil umgewandelt, welches seinem Basen-Paarungsverhalten dem Thymidin, mithin einer anderen Base entspricht. 5-Methylcytosin wird nicht modifiziert. Damit wird die ursprüngliche DNA so umgewandelt, daß Methylcytosin, welches ursprünglich durch sein Hybridisierungsverhalten vom Cytosin nicht unterschieden werden kann, jetzt durch "normale" molekularbiologische Techniken nachgewiesen werden kann. Alle diese Techniken beruhen auf Basenpaarung, welche jetzt voll ausgenutzt werden kann. Der Stand der Technik was die Empfindlichkeit betrifft wird durch ein Verfahren definiert, welches die zu untersuchende DNA in einer Agarose-Matrix einschließt, dadurch die Diffusion und Renaturierung der DNA (Bisulphit reagiert nur an einzelsträngiger DNA) verhindert und alle Fällungs- und Reinigungsschritte durch schnelle Dialyse ersetzt (Olek, A. et al., Nucl. Acids. Res. 24, 5064-5066). Mit dieser Methode können einzelne Zellen untersucht werden, was das Potential der Methode veranschaulicht. Allerdings werden bisher nur einzelne Regionen bis etwa 3000 Basenpaare Länge untersucht, eine globale Untersuchung von Zellen auf Tausende von möglichen Methylierungsereignissen ist nicht möglich. Allerdings kann auch dieses Verfahren keine sehr kleinen Fragmente aus geringen Probenmengen zuverlässig analysieren. Diese gehen trotz Diffusionsschutz durch die Matrix verloren.

### 2.8. Stand der Technik bei der Anwendung der Bisulphit-Technik

Die Bisulphit-Technik wird bisher bis auf wenige Ausnahmen (z.B. Zeschnigk, M. et al., Eur. J. Hum. Gen. 5, 94-98; Kubota T. et al., Nat. Genet. 16, 16-17) nur in der Forschung angewendet. Immer aber werden kurze, spezifische Stücke eines bekannten Gens nach einer Bisulphit-Behandlung amplifiziert und entweder komplett sequenziert (Olek, A. and Walter, J., Nat. Genet. 17, 275-276) oder einzelne Cytosin-Positionen durch eine "Primer-Extension-Reaktion" (Gonzalgo, M. L. and Jones, P.A., Nucl. Acids. Res. 25, 2529-2531) oder Enzymschnitt (Xiong, Z. und Laird, P.W., Nucl. Acids. Res. 25, 2532-2534) nachgewiesen. Alle diese Referenzen stammen aus dem Jahre 1997. Das Konzept, komplexe Methylierungsmuster zur Korrelation mit phänotypischen Daten komplexer genetischer Erkrankungen zu verwenden, geschweige denn über einen Auswertealgorithmus wie zum Beispiel ein neuronales Netzwerk auszuwerten, ist in der Literatur bisher nicht erwähnt und ist auch nach dem Stand der Technik methodisch nicht durchführbar.

### 3. Aufgabe der Erfindung und Lösung der Aufgabe

Zusammenfassend weist der Stand der Technik Schwächen auf, welche durch das erfindungsgemäße Verfahren gelöst werden.

Die Aufgabe wird dadurch gelöst, daß ein Verfahren zur Charakterisierung, Klassifizierung und Unterscheidung von Geweben und Zelltypen, zur Vorhersage des Verhaltens von Geweben und Gruppen von Zellen und zur Identifizierung von in ihrer Expression veränderten Genen zur Verfügung gestellt wird, wobei
in einer aus einer beliebigen Gewebeprobe gewonnenen unbehandelten, gescherten oder mittels einer Restriktions-Endonuklease gespaltenen genomischen DNA auf an sich bekannte Art und Weise die Base Cytosin, aber nicht 5-Methylcytosin durch Behandlung mit einer Bisulphit-Lösung in Uracil umgewandelt wird,

Fraktionen der so behandelten genomischen DNA durch Verwendung von unspezifisch hybridisierenden Oligonukleotiden und/oder von einer Mischung aus zwei oder mehr verschiedenen, spezifisch hybridisierenden Oligonukleotiden und/oder von solchen Oligonukleotiden, welche zu - vor der Bisulphit-Behandlung an die Enden der gespaltenen DNA ligierten - Adaptor-Oligonukleotiden komplementär sind, amplifiziert werden,
man insgesamt eine solche Menge der verbleibenden Cytosine auf dem Guanin-reichen DNA-Strang und/oder Guanine auf dem Cytosin-reichen DNA-Strang aus den amplifizierten Fraktionen durch eine Hybridisierung oder Polymerase Reaktion nachweist, daß man die bei einer solchen Analyse generierten und automatisch an einen Verarbeitungsalgorithmus übertragenen Daten analysiert und auf den Phänotyp des analysierten Zellmaterials rückschließt.

Die unspezifisch hybridisierenden Oligonukleotide sind entweder sehr kurz oder degeneriert, d.h. an einer Position des Oligonukleotids wird mehr als eine Base mit ähnlicher Effizienz gekoppelt. Die Mischung aus zwei oder mehr verschiedenen, spezifisch hybridisierenden Oligonukleotiden bewirkt ebenfalls, dass an den voneinander verschiedenen Positionen eine Degenerierung erfolgt, d.h. mehr als eine Base mit ähnlicher Effizienz gekoppelt wird.

Erfindungsgemäß vorteilhaft ist es, daß
die aus dieser Analyse gewonnenen Daten mehrerer oder vieler solcher Versuche an DNA-Proben aus phänotypisch gleichen oder ähnlichen Zellen oder Geweben in einer Trainingsphase über ein neuronales Netzwerk oder andere Auswertealgorithmen mit dem Phänotyp der Zellen, deren DNA untersucht wurde, korreliert werden,
die bei dieser Trainingsphase in den Auswertealgorithmus aufgenommenen Daten über den Zusammenhang zwischen Phänotyp und Methylierungsmuster dazu benutzt werden, durch Generierung eines Methylierungsmusters einer DNA-Probe unbekannten Ursprungs den Phänotyp der Zellen, deren DNA untersucht wurde, abzuleiten oder
die bei dieser Trainingsphase in den Auswertealgorithmus aufgenommenen Daten über das Methylierungsmuster der DNA eines bekannten Zelltyps dazu benutzt werden, solche Cytosin-Positionen zu identifizieren, welche in der untersuchten DNA von dem in der Trainingsphase ermittelten Methylierungszustand abweichen.

Es ist ferner erfindungsgemäß vorteilhaft, daß die DNA vor der Behandlung mit Bisulphit mit solchen Restriktionsendonukleasen gespalten wird, welche Cytosin im Kontext 5'-CpG-3' in ihrer Erkennungssequenz enthalten und die DNA nur an solchen dieser Erkennungssequenzen spalten, in denen Cytosin im Kontext 5'-CpG-3' an der 5'-Position unmethyliert vorliegt.

Erfindungsgemäß vorteilhaft ist es weiterhin, daß
bevor die genomische DNA auf an sich bekannte Art und Weise mit einer Bisulphit Lösung modifiziert wird, diese genomische DNA mit einer Restriktionsendonuklease gespalten wird,
die entstehenden Enden durch eine Ligationsreaktion mit bekannten, kurzen und doppelsträngigen, auch Adaptoren genannten DNA-Sequenzen versehen werden,

Oligonukleotide, die gegen Bisulphit-behandelte Adaptoren komplementär sind, dazu verwendet werden, alle auf diese Art entstandenen DNA-Fragmente oder Sub-Populationen aus der Gesamtheit aller auf diese Art entstandenen Fragmente nach einer Behandlung mit Bisulphit, zu amplifizieren.

Bevorzugt ist es dabei, daß die Reaktion einer genomischen DNA-Probe mit einer Bisulphit-Lösung zwecks Umwandlung von Cytosinen zu Uracilen bei gleichzeitiger Erhaltung von Methylcytosin unter zyklischer Variation der Reaktionstemperatur bei Temperaturen zwischen 0 °C und 100 °C stattfindet.

Bevorzugt ist es auch, daß die DNA-Probe vor der Behandlung mit Bisulphit in eine temperierbare, nur für kleine Moleküle durchlässige poröse Kapillare, in welcher die folgenden Reaktionsschritte der Bisulphitbehandlung durch Zu- und Abfuhr der Reagenzien durch Dialyse ausgeführt werden können.

Es ist weiterhin erfindungsgemäß bevorzugt, daß die DNA-Probe vor der Behandlung mit Bisulphit in eine temperierbare, für kleine Moleküle undurchlässige Kapillare überführt wird, in welcher die folgenden Reaktionsschritte der Bisulphitbehandlung durch Zu- und Abfuhr der Reagenzien durch Zuleitung von Reagenzien durch angeschlossene Kapillare ausgeführt werden können.

Erfindungsgemäß vorteilhaft ist es außerdem, daß die sich an die Bisulphitbehandlung anschließenden Polymerase-Reaktionen in derselben Kapillare wie die Bisulphitbehandlung oder einer sich an diese Kapillare anschließenden Kapillare oder einem an diese Kapillare angeschlossenen Behälter durchgeführt werden.

Vorteilhaft ist es auch, daß in einer Kapillare, in welcher Polymerase-Reaktionen mit einer mit Bisulphit-behandelten DNA-Probe erfolgt auch eine Längentrennung der entstehenden Fragmentpopulation durchführt wird.

Bevorzugt ist ferner, daß eine behandelte DNA durch Fällung des Bisulphit von diesem getrennt wird.

Es ist weiterhin erfindungsgemäß bevorzugt, daß für die Amplifikation der Bisulphit-behandelten genomischen Proben-DNA Oligonukleotide zweier Klassen kombiniert werden, wobei die Oligonukleotide der einen Klasse die Base Cytosin oder deren Analoge nicht, ausschließlich im Kontext 5'-CpG-3', in nur sehr geringem, die Amplifikation nicht beeinflussendem Maße oder nur in die Amplifikation nicht beeinflussenden Bereichen der Oligonukleotide enthalten und wobei die Oligonukleotide der anderen Klasse die Base Guanin oder deren Analoga nicht, ausschließlich im Kontext 5'-CpG-3', in nur sehr geringem, die Amplifikation nicht beeinflussendem Maße oder nur in die Amplifikationnicht beeinflussenden Bereichen, wie zum Beispiel 5'-Bereichen der Oligonukleotide enthalten und wobei beide Klassen von Oligonukleotiden entweder
a) so kurz sind, daß die in einer Amplifikation mit nur je einem Vertreter beider Klassen mehr als hundert verschiedene Fragmente amplifiziert werden oder
b) diese Oligonukleotide so viele degenerierte Positionen enthalten, daß in einer Amplifikation mit nur je einem Vertreter beider Klassen mehr als hundert verschiedene Fragmente amplifiziert werden oder
c) so viele Vertreter beider Klassen von Oligonukleotiden in einer Amplifikation verwendet werden, daß mehr als hundert verschiedene Fragmente amplifiziert werden.

Vorteilhafterweise ist es optional vorgesehen, daß die behandelte und amplifizierte DNA in separaten Ansätzen zum Zwecke einer Polymerase-Reaktion mit in jeder Reaktion unterschiedlichen Oligonukleotiden gemischt wird, welche
an deren 5'-Termini zu den Adaptoren oder generell für die Amplifikation der Bisulphit-behandelten Oligonukleotide komplementär sind und
welche an deren 3'-Termini in jeder Reaktion unterschiedlich sind und
deren variable 3'-Termini hinter der bekannten Adaptor-Sequenz oder Oligonukleotid-Sequenz beginnen
und deren variable 3'-Termini über die bekannte Adaptor-Sequenz zwischen zwei und zwölf Nukleotide in die unbekannte Templat-DNA Sequenz hineinreichen.

Dabei ist es wiederum besonders bevorzugt, daß solche Reaktionen, in welchen eine Polymerasereaktion mit Oligonukleotiden gestartet wird, welche zu einer mit Bisulphit behandelten DNA komplementär sind, außer den drei Nukleotiden dATP, dTTP und dCTP oder Analogen dieser drei Nukleotide
ein zur Base Cytosin komplementäres Nukleotid-Analog enthalten, welches nach Einbau durch die Polymerase jede weitere Strang-Verlängerung blockiert oder
gar kein zur Base Cytosin komplementäres Nukleotid oder Nukleotid-Analog enthalten.

Weiterhin ist dabei erfindungsgemäß bevorzugt, daß solche Reaktionen, in welchen eine Polymerasereaktion mit Oligonukleotiden gestartet wird, welche zu einer mit Bisulphit-behandelten DNA komplementären DNA komplementär sind außer den drei Nukleotiden dATP, dTTP und dGTP oder Analogen dieser drei Nukleotide
ein zur Base Guanin komplementäres Nukleotid-Analog enthalten, welches nach Einbau durch die Polymerase jede weitere Strang-Verlängerung blockiert oder
gar kein zur Base Guanin komplementäres Nukleotid oder Nukleotid-Analog enthalten.

Ganz besonders bevorzugt ist es dabei auch, daß die Termination einer Polymerase-Reaktion an den Stellen, an denen vormals Methylcytosin in der DNA-Probe enthalten war durch modifizierte Terminatoren vonstatten geht, und die Detektion der spezifisch terminierten Polymerase-Reaktionsprodukte über die Detektion der Modifikation erfolgt.

Weiterhin ist erfindungsgemäß vorgesehen, daß die verschiedenen, aus einer geeigneten Kombination resultierende Fragmentgemische der einzelnen Reaktionsansätze auf individuelle Punkte der Ionenquelle eines MALD1-TOF oder anderen Massenspektrometers aufgetragen werden und die Fragment-Zusammensetzungen der einzelnen Reaktionen durch Massebestimmung aller DNA-Fragmente bestimmt werden.

Ferner ist es bevorzugt, daß die verschiedenen, aus einer geeigneten Kombination resultierende Fragmentgemische der einzelnen Reaktionsansätze auf individuelle Bahnen einer Gelelektrophorese aufgetragen werden und die Fragment-Zusammensetzungen der einzelnen Reaktionen durch Längenmessung aller DNA-Fragmente bestimmt werden.

Vorteilhafterweise ist es erfindungsgemäß vorgesehen, daß die erfindungsgemäß definierten Oligonukleotide, mit denen Polymerase-Reaktionen gestartet werden, mit je Oligonukleotid unterschiedlicher Sequenz unterschiedlichen chemischen Markierungen gekoppelt sind und die Detektion und Unterscheidung der Polymerase-Reaktionsprodukte durch Analyse der chemischen und/oder physikalischen Eigenschaften der verschiedenen Markierungen mit gängigen chromatographischen oder massenspektrometrischen Verfahren erfolgt.

Hierbei ist es insbesondere vorteilhaft, daß
die im ersten Amplifikationsschritt hergestellte Fragment-Fraktion der zu untersuchenden, Bisulphit-behandelten DNA mit zwei oder mehr chemisch unterschiedlich markierten Oligonukleotiden gleichzeitig gemischt wird,
diese Oligonukleotide in einem Reaktionsansatz als Primer für eine Polymerasereaktion benutzt werden,
die entstehende komplexe Mischung von Fragmenten in einem ersten analytischen Schritt einer elektrophoretischen Längentrennung unterzogen wird und
die einzelnen Längenfraktionen der aus der aus der Elektrophorese resultierenden Fragmentgemische einer chromatographischen oder massenspektrometrischen Analyse unterzogen werden, welche in jeder Längenfraktion die Präsenz oder Absenz der die Oligonukleotide charakterisierenden chemischen Markierungen detektiert.

Es ist erfindungsgemäß ferner vorgesehen, daß auf eine Oberfläche Oligonukleotide aufgebracht werden, welche
entweder die Base Cytosin oder deren Analoge nicht, nur im Kontext 5'-CpG-3' oder nur in die Hybridisierung mit einer Proben-DNA nicht beeinflussenden Bereichen enthalten
oder die Base Guanin nicht, nur im Kontext 5'-CpG-3' oder nur in die Hybridisierung mit einer Proben-DNA nicht beeinflussenden Bereichen enthalten.

Dabei ist es erfindungsgemäß bevorzugt, daß die Bisulphit-behandelte und erfindungsgemäß amplifizierte Proben-DNA
mit auf einer Oberfläche fixierten Oligonukleotiden hybridisiert wird, welche in an sich bekannter Art und Weise so auf dieser Oberfläche fixiert sind, daß an jedem Punkt der Oberfläche bekannt ist, welche Oligonukleotid-Sequenz sich an genau diesem Punkt befindet, - eine Hybridisierung der amplifizierten Proben-DNA mit den fixierten Oligonukleotiden nur dann erfolgt oder nach geeigneten Waschschritten bestehen bleibt, wenn Oligonukleotide und Proben-DNA in den für eine Hybridisierung wesentlichen Bereichen vollständig komplementär sind.

Das Verfahren löst die Aufgabe, eine extrem große Menge an Parametern zu ermitteln, welche für das Verhalten von Zellen diagnostisch sind. Dafür muß sowohl ein völlig neues Konzept zur Analyse von Zellen erarbeitet, völlig neue Auswertemechanismen an dieses geknüpft werden und weiterhin die technische Basis für die Generierung der Daten bereitgestellt werden. Das Verfahren nutzt zum ersten Mal potentielle Informativität der Cytosin-Methylierung aus und stellt die dafür notwendigen analytischen Verfahren und damit verbundenen Auswertealgorithmen zur Verfügung. Das erfindungsgemäße Verfahren dient daher dazu, bei von erblichen Defekten betroffenen Zellen solche sekundär involvierten Gen-Loci ausfindig zu machen, welche durch Methoden nach dem Stand der Technik theoretisch nicht oder aber nur sehr schwer ermittelt werden könnten: Das Verfahren weist genetisch veränderte Loci nach, deren (daher möglicherweise epi-) genetische Veränderungen keine wirkliche Änderung der Basensequenz beinhaltet. Auf diesem Wege legt das erfindungsgemäße Verfahren Ziele für neue therapeutische Strategien offen. Das Verfahren löst weiterhin die Aufgabe, entartete Zellen so zu klassifizieren, daß wesentlich mehr und genauere Korrelationen zwischen (Epi-)Genotyp und Phänotyp geschaffen werden, als nach dem Stand der Technik möglich sind. Das erfindungsgemäße Verfahren ermöglicht es darüber hinaus, eine Vorhersage über das wahrscheinliche zukünftige Verhalten entarteter Zellen und die Reaktion solcher Zellen auf Stimuli von innerhalb und außerhalb des Körpers zu treffen. Es leistet damit letztendlich auch Hilfestellung bei der Auswahl der besten Therapieansätze bei Krebserkrankungen. Weiterhin ermöglicht es das Verfahren, die genetischen und/oder biochemischen Gemeinsamkeiten von Tumorzellen zu ermitteln, welche phänoty pisch ähnlich aber genotypisch (soweit nach dem Stand der Technik feststellbar) unterschiedlich sind. Die Vermutung, auf der dieser Anspruch des Verfahren basiert, beinhaltet, daß verschiedenste Genotypen zu sehr ähnlichen Epigenotypen und damit zu sehr ähnlichen Phänotypen führen könnten. Damit ist das vorgeschlagene Verfahren auch in der Lage, solche Veränderung der Genexpression von Tumorzellen zu detektieren, welche nicht, oder nur indirekt durch Veränderungen der Basensequenz verursacht werden.

### 4. Ausführliche Beschreibung der Lösung der Aufgabenstellung durch das erfindungsgemäße Verfahren

Das vorgeschlagene Verfahren löst die beschriebene Aufgabenstellung auf innovative Art und Weise durch Kombination und Verbesserung verschiedener Verfahren des Standes der Technik. Bestimmte erfindungsgemäße Modifizierungen dieser an sich bekannten Verfahren dienen dazu, diese an die neuen Anforderungen anzupassen, so daß ein völlig neues Gesamtverfahren entsteht, welches im folgenden an Hand von bevorzugten Verfahrensvarianten beschrieben und durch Beispiele ausgeführt wird.

### 4.1. Vorbehandlung der DNA-Probe vor der Behandlung durch eine Bisulphit-Lösung

Grundsätzliche Verfahrensschritte, wie zum Beispiel die Isolation von Gewebe oder Zellen und die Extraktion von DNA aus diesen, finden in an sich bekannter Art und Weise statt. Die Extraktion der DNA für die weitere Analyse wird allerdings bei den bevorzugten Verfahrensvarianten in einem sehr kleinen Volumen stattfinden,meist, wie die eigentliche Behandlung mit Bisulphit selber, in einer Schicht von Öl, welche allen Kontakt zur Außenwelt verhindert. Dies dient dazu die Verluste an DNA so gering zu halten, daß auch bei kleinsten Ausgangsmengen ein reproduzierbares Ergebnis gewährleistet ist. Die Extraktion der DNA aus Zellen oder Geweben kann auch direkt in einer wie unten beschriebenen Kapillare stattfinden, in der dann auch alle Folge-Reaktionen durchgeführt werden können. Eine Beschränkung des Extraktionsvolumens ist allerdings kein notwendiger Bestandteil des vorgeschlagenen Verfahrens.

Extrahierte DNA kann nun unbehandelt der Bisulphit-Behandlung zugeführt werden, geschert, oder mit Restriktionsendonukleasen spezifisch gespalten werden.

Das erfindungsgemäße Verfahren kann an diesem Punkt in zwei unterschiedliche Verfahrensvarianten unterteilt werden. Eine Variante, innerhalb derer die letztendliche Detektion der einzelnen Methyl-Cytosin Positionen durch eine Hybridisierung mit Oligonukleotiden durchgeführt wird, bedarf an diesem Punkt normalerweise keiner weiteren Vorbehandlung der DNA. Eine zweite Variante, dadurch gekennzeichnet, daß die genomweite Amplifikation der DNA-Probe durch Oligonukleotide erfolgt, welche gegen an die Enden der DNA ligierte, mit Bisulphit behandelte Adaptoren komplementär sind, bedarf der Ligation von solchen Adaptoren an die einzelnen Fragmente der gespaltenen DNA. Die Adaptoren sind kurze, doppelsträngige DNA-Moleküle, die in der Regel an einem Ende einen einzelsträngigen Überhang aufweisen. Dieser Überhang ist zu den Enden der geschnittenen DNA-Probe komplementär, so daß an beide Enden der DNA-Fragmente der Probe ein solcher Adaptor mittels einer geeigneten Ligase angebracht werden kann. Dazu werden solche Mengen von Adaptoren hinzugegeben, daß diese in Relation zur Zahl der Fragment-Enden im Überschuß vorliegen. Die Ligationen von Adaptoren an Proben-Fragmente können aber im Prinzip auch ohne komplementäre einzelsträngige Überhänge durchgeführt werden. Die Einzelnen Reaktionen sind dabei im Prinzip Stand der Technik (Sambrook et al. Molecular Cloning: A laboratory manual, CSHLP, 1989), sollen also nicht weiter ausgeführt werden. Die Kombination der Ligation von Adaptoren mit Bisulphit-Behandlung und nachfolgender genomweiter Amplifikation ist prinzipiell innovativ und in Literatur und Patentliteratur nicht erwähnt.

### 4.2. Erfindungsgemäße Modifikationen der Bisulphit-Methode

Grundlage aller erfindungsgemäßen Verfahrensvarianten ist die Methode der Modifikation von einzelsträngiger DANN durch Bisulphit. Um einige der erfindungsgemäßen Verfahrensvarianten zu ermöglichen, sind allerdings einige Modifikationen der Bisulphit-Methode erforderlich.

Hauptsächliche Varianten dieser Methode beruhen zum einen auf der Tatsache, daß nicht nur sehr kleine Gesamtmengen von Ausgangsmaterial benutzt werden sollen (im Grenzfall nur eine oder einige zehn Zellen), sondern einige Verfahrensvarianten auch die Verwendung sehr kleiner Fragmente erfordern. Zum anderen ist es für eine routinemäßige Anwendung des erfindungsgemäßen Verfahrens in der klinischen Diagnostik notwendig, alle Verfahrensschritte so zu automatisieren, daß ein möglichst hoher Grad an Reproduzierbarkeit erreicht werden kann.

Alle Schritte der Bisulphit-Methode sollen daher in sehr kleinem Volumen unter völlige Abschottung von der "Außenwelt" stattfinden. Der Einschluß der Bisulphit Reaktion in eine Agarose-Matrix stellt dabei schon einen Fortschritt hinsichtlich der Diffusion von Fragmenten dar, trotzdem findet die Reaktion immer noch in einem sehr großen Volumen wäßriger Bisulphit-Lösung statt. Dadurch können kleine, wichtige DNA Fragmente in die Lösung diffundieren und gehen somit für die weitere Analyse verloren.

Teil des erfindungsgemäßen Verfahrens ist die Ausführung der Bisulphit-Methode unter Verzicht auf jegliches Außenvolumen. Die Bisulphit-Reaktion wird zum Beispiel unter Oel in einem Volumen von nur 1 ml bis 10 ml durchgeführt, alle Bestandteile können so direkt von einem Roboter unter das Oel pipetiert werden und formen dort einen einzelnen Tropfen, in dem alle weiteren Reaktionsschritte vonstatten gehen. Die Schwierigkeit der Herstellung einer Bisulphit-Lösung mit Konzentrationen, wie sie nach dem Stand der Technik erforderlich sind, und die Tatsache, daß die Lösung dieses Dilemmas nach Stand der Technik durch Verwendung niedrigerer Reaktionszeiten bei geringerer Bisulphit-Konzentration zu signifikanten Schäden an der DNA-Probe führt, werden durch das erfindungsgemäße Verfahren gelöst.

Dieses Verfahren nutzt die Tatsache, daß die verschiedenen Reaktionsschritte der Bisulphit-Reaktion Gleichgewichtsreaktionen sind. Diese Gleichgewichte liegen bei den zwei wichtigen Reaktionsschritten. der Sulphonierung des Cytosin und der nachfolgenden Deaminierung. bei unterschiedlichen Temperaturen auf der richtigen (sulphonierten und deaminierten) Seite. Berücksichtigt man die Kinetiken, mit denen sich die jeweiligen Gleichgewichte einstellen, so erweist es sich als nützlich die Bisulphit-Reaktion unter zyklischen Bedingungen, bei wechselnden Temperaturen durchzuführen. Eine bevorzugte Verfahrensvariante beinhaltet einen Wechsel von 4 °C (10 min) auf 50° (20 min). Alle anderen Temperaturen und Reaktionszeiten bei bestimmten Temperaturen sollen aber in das erfindungsgemäße Verfahren eingeschlossen sein. Zum Beispiel kann es sich unter bestimmten Bedingungen als vorteilhaft erweisen, wenn wesentlich kürzere Reaktionszeiten eingestellt werden. Es ist auch nützlich und grundsätzlich neuartig, zwischen einen Deaminationsschritt (bei hoher Temperatur, >=50 °C) und einen darauffolgenden erneuten Sulphonierungsschritt einen Schritt einzufügen, bei dem die zu untersuchende DNA erneut bei sehr hoher Temperatur denaturiert wird. Denaturierungstemperaturen liegen in der Regel für hochmolekulare DNA bei >90 °C, können aber innerhalb des zu schützenden Verfahrens auch darunter liegen. Dies hat zwei Gründe. Einerseits gibt es Verfahrensvarianten, bei denen sehr kurze DNA-Fragmente untersucht werden. Zu anderen sinkt in jedem Reaktionszyklus durch erfolgte Konvertierung von Cytosinen zu Uracilen die Komplementarität zwischen den Strängen. Daher kann ein zyklisches Reaktionsprotokoll sehr komplex aussehen. Die Denaturierungstemperatur kann zum Beispiel in den ersten Zyklen bei über 90 °C liegen, in späteren Zyklen aber niedriger reguliert werden. Mehrstufige Reaktionen können in allen Aspekten nur durch extrem aufwendige Testreihen optimiert werden. Der beantragte Schutz soll sich daher allgemein auf zyklisch durchgeführte Bisulphit-Reaktionen beziehen.

Eine weitere Lösung der oben erwähnten Probleme mit dem Stand der Technik beruht auf der Verlegung eines oder mehrerer Schritte des Verfahrens in eine Kapillare. Dabei gibt es prinzipiell zwei Varianten. Die Kapillare kann 1) dicht sein und 2) nach der Art eines sehr dünnen Dialyseschlauches für gewisse Lösungsmittel durchlässig sein.

Die Variante nach 1) beinhaltet, daß ein, wie in den obigen Beispielen beschriebener Tropfen mit DNA, Bisulphit und Radikalfänger in wäßriger Lösung durch eine von außen beheizbare und kühlbare Kapillare geführt wird. Der Tropfen kann dabei durch eine Flüssigkeit oder Gasphase innerhalb der Kapillare isoliert sein. Alle Reaktionen finden dann innerhalb dieser Kapillare statt, zusätzliche Reagenzien können durch Einmündungen hinzugegeben werden. Da diese Kapillare nach Variante 1) nach außen hin völlig abgeschlossen ist, muß allerdings für nachfolgende Reaktionsschritte eine Matrix-Lösung hinzugegeben werden, welche die oben genannten Probleme hervorruft und erfindungsgemäße Lösungen benötigt.

Die Variante nach 2) beinhaltet, daß zunächst nur die DNA Lösung, nach entsprechender Vorbehandlung durch erfindungsgemäße oder andere Verfahrensschritte durch die poröse Kapillare geführt wird. Die Kapillare selber wird durch Behälter mit Lösungen geführt, welche für Reaktionsschritte innerhalb der Kapillare benötigt werden. Konkret wird die DNA Lösung innerhalb der Kapillare bei dieser Variante zunächst durch eine Bisulphit-Lösung geführt, welche zusätzlich entweder zyklisch temperiert werden kann oder bei konstanter Temperatur vorliegt. In einem weiteren Schritt wird nach erfolgter Bisulphit-Reaktion die Kapillare durch eine Dialyse-Lösung, dann durch eine alkalische Lösung und schließlich durch eine weitere Dialyse-Lösung geführt. Nach diesen Schritten der Bisulphit-Behandlung in der Kapillare können, dies soll eine weitere Verfahrensvariante darstellen, auch alle weiteren PCR und Primer-Extensions-Schritte in der selben Kapillare durchgeführt werden. In dem Fall, daß die verschiedenen Primer für die Primer-Extension erfindungsgemäß durch eine besondere chemische Modifizierung markiert sind, so kann auch direkt an alle diese PCR und Primer-Extensions-Schritte in einer Verlängerung derselben Kapillare eine Kapillarelektrophorese ausgeführt werden. Diese trennt die Extensionsprodukte der Länge nach, eine nachfolgende massenspektrometrische oder chromatographische oder optische Analyse trennt dann die gesammelten Größenfraktionen nach ihrer Markierung hin auf und generiert damit in der zweiten Analysedimension das Ergebnis-Spektrum oder Ergebnis-Chromatogramm.

Die Verwendung einer Kapillare für die Bisulphit und PCR und/oder Extensions-Reaktionen erleichtert auch die Verwendung einer anderen der erfindungsgemäßen Detektionsvarianten. Die Fragmente können nämlich direkt nach der Amplifikation in eine Kapillare geleitet werden, welche, wie weiter unten beschrieben, die für die einzelnen Methylcytosin spezifischen Oligonukleotide als Hybridisierungspartner auf der Innenseite trägt.

Eine weitere Verfahrensvariante hierzu beruht auf einer anderen Eliminierung der hochmolare Bisulphit-Lösung, als durch Dialyse. Die Vorteile dieser Variante eliminieren einen weiteren Nachteil der bisher beschriebenen Varianten.

Jede Dialyse in Agarose läßt Teile der Prozedur in einem großen Volumen wäßriger Lösung stattfinden. Dabei droht ein Verlust an DNA-Fragmenten durch Diffusion. Ein Problem bei Varianten, welche in einer Kapillare stattfinden ist, daß ein geringer, aber bei kleinsten Mengen DNA eventuell signifikanter Prozentsatz an DNA-Fragmenten an die Innenwandung der Kapillare bindet und so für die Analyse verloren gehen kann.

Daher wird folgendes Verfahren vorgeschlagen: Die DNA-Extraktion wird, wie beschrieben, in einem sehr kleinen Volumen unter einer Ölschicht vorgenommen. In der bevorzugten Verfahrensvariante handelt es sich um ein Volumen von 1 µl. Natürlich wird das Verfahren von Verwendung kleinerer oder größerer Volumina nicht wesentlich verändert. Also fallen diese auch in den beantragten Schutzbereich. Die DNA wird denaturiert (wie zitiert). Die nötige Bisulphit-Konzentration wird dann durch Zugabe eines größeren Volumens einer Bisulphit-Lösung (beispielsweise von 4 µl) zugegeben, welche etwas größer ist, als für die eigentliche Behandlung notwendig ist, so daß sich die benötigte Endkonzentration und pH automatisch unter dem Öl einstellen. Daraufhin wird die Bisulphit-Reaktion auf eine der beschriebenen Arten durchgeführt.

Im nächsten Verfahrensschritt wird (in einer erfindungsgemäß bevorzugten Verfahrensvariante) eine gleiche Molmenge eines Salzes, zum Beispiel Barium-Hydroxid in Lösung hinzugegeben, dessen Kation mit dem Bisulphit ein unlösliches Salz bildet und somit aus der Lösung ausfällt. Die Zugabe dieser Lösung bewirkt außerdem einen Anstieg des pH auf Werte, bei denen die Desulphonierung der in den ersten Reaktionsschritten sulphonierten und deaminierten Cytosine stattfinden kann. Während der Desulphonierungs-Reaktion, welche sehr schnell abläuft, kann das ausgefallene Bisulphit-Salz durch kurze Zentrifugation von der wäßrigen Probenlösung getrennt werden. Es wird aber vorgezogen, ein Salz zu verwenden, welches die folgenden Eigenschaften hat. Das Kation bildet mit dem Bisulphit ein Salz, welches auch unter den Bedingungen des Amplifikations-Prozesses unlöslich bleibt und den Amplifikations-Prozeß in keiner Weise nachteilig beeinflußt. Auch darf keines der Ionen, welche bei einem solchen Prozeß nicht aus der Lösung ausfallen, in den Mengen, in denen solche Ionen dann vorliegen, den Amplifikations-Prozeß stören. Die mögliche Störwirkung solcher Salze im Amplifikations-Prozeß kann aber auch umgangen werden, indem extrem präzise angesetzte Salzlösungen verwendet werden, welche mit ebenso extremer Genauigkeit pipettiert werden können. Die Verwendung identischer Mengen von Salzen führt zu einer quantitativen Eliminierung der potentiell störenden Ionen. Die Verwendung von Kalium Bisulphit oder anderer Gegen-Ionen zum Bisulphit, die mit den nachfolgenden Amplifikationspuffern komplementär erleichtern auch das im Folgenden beschriebene Umpuffern für die Amplifikations-Reaktion.

Im nächsten Verfahrensschritt wird dann ein weiteres Volumen einer Lösung unter das Öl gegeben, welche die folgenden Eigenschaften hat. Die Salzkomposition ist derart, daß bei Mischen mit der unter Öl vorliegenden Lösung der behandelten DNA solche Salzkonzentrationen und pH entstehen, die einen enzymatischen Amplifikations-Prozeß erlauben. Dabei können alle thermostabilen Polymerasen jeden Ursprungs verwendet werden. Die Art der verwendeten Polymerase ist unwesentlich, kann auch entsprechend den vorherrschenden Pufferbedingungen variiert werden und es soll daher ein Schutz für die Verwendung aller solcher Polymerasen beantragt werden. Zweitens ist eine solche Polymerase, alle Nukleotide und die benötigten Oligonukleotid-Primer in dieser Lösung enthalten. Nach Zugabe dieser Lösung kann also direkt im selben Reaktionsgefäß eine Amplifikation erfolgen. Damit ist jeder Kontakt mit der "Außenwelt" während der gesamten Verfahrensabläufe unmöglich; es kann keine noch so kleine Probenmenge verloren gehen.

### 4.3. Genomweite generische Amplifikationen Bisulphit-behandelter DNA

Die Detektion Tausender bis Millionen von Methylcytosin-Positionen erfordert in jedem Falle eine Amplifikation eines großen Prozentsatzes aller möglichen Sequenzen eines Proben-Genoms. Dieser Teil des erfindungsgemäßen Verfahrens soll, wie schon im Absatz "Vorbehandlung", in zwei prinzipiell unterschiedliche Varianten unterteilt werden.

Die erste Variante dieses Verfahrensschrittes beruht auf der Ligation von Adaptoren an die fragmentierte DNA vor der Bisulphitbehandlung. In der einfachsten Form wird hierfür ein Oligonukleotid verwendet, welches gegen die Adaptor-Sequenzen komplementär ist, so wie sie nach einer Bisulphitbehandlung vorliegen. Dabei kann dieses Oligonukleotid mit jedem beliebigen Bereich der Adaptor-Sequenz hybridisieren. Dies führt im Falle einer Polymerase-Reaktion mit diesen Komponenten theoretisch zu einer Amplifikation aller Fragmente mit Adaptoren an beiden Enden. Beispielsweise könnten dies alle Fragmente sein, die eine vorherige Spaltung mit einer Restriktionsendonuklease ergibt. Für einige Verfahrensvarianten ist es aber, auf Grund der limitierten Zahl der einzelnen Fragmente, die eine solche Amplifikation produziert, notwendig, nach einer kleinen Anzahl von Amplifikationszyklen die Reaktion in verschiedene Teilreaktionen zu unterteilen. Diese Teilreaktionen können nun mit Oligonukleotiden durchgeführt werden, welche einige über die eigentliche Adaptorsequenz hinaus, nämlich zwischen einer und vier Basen, in die unbekannte Sequenz der verschiedenen Fragmente hineinreichen. Die Oligonukleotide der verschiedenen Reaktionen werden so gewählt, daß jedes einen solchen Teil aller möglichen unbekannten Sequenzen abdeckt, daß die Gesamtheit aller dieser Oligonukleotide in den verschiedenen Reaktionen alle möglichen Sequenzen abdeckt, die theoretisch hinter den bekannten Adaptorsequenzen vorliegen können. Zum Beispiel können vier Reaktionen angesetzt werden, wobei das Oigonukleotid der ersten Reaktion am 3'-Terminus hinter der bekannten Adaptor-komplementären Sequenz die Base Adenin enthält, die zweite ein Cytosin, die dritte Guanin und die vierte Thymidin. Dieses Prinzip kann natürlich auch mit mehr als vier verschiedenen Reaktionen durchgeführt werden, wobei dann die Sequenz am 3'-Terminus der Oligonukleotide mehr als eine Base beträgt. Dabei können Positionen am 3'-Terminus der Oligonukleotide auch sogenannte degenerierte Positionen aufweisen. Dies bedeutet, daß an einer Position mehr als eine Base mit ähnlicher Effizienz an das Oligonukleotid gekoppelt wird oder zwei oder mehr Oligonukleotide mit nichtdegenerierter Sequenz gemischt werden. Damit lassen sich alle möglichen Sequenzen in Gesamtzahlen von Reaktionen abdecken, welche nicht Potenzen der Zahl vier sind.

Auf diese Art kann in jeder Reaktion eine Subpopulation aller Fragmente amplifiziert werden, was eine höhere Verläßlichkeit und höhere Amplifikation der einzelnen Fragmente ermöglicht. Im Prinzip ist auch eine schrittweise Aufteilung der Reaktionen möglich, so daß eine erste Zahl von Amplifikationszyklen mit nur einem, alle Sequenzen abdeckenden Oligonukleotid durchgeführt wird, die Reaktion daraufhin auf zum Beispiel auf vier Reaktionen mit pro Reaktion einer spezifischen 3'-Base aufgeteilt wird und sich einige weitere Amplifikationszyklen, gefolgt von einer oder mehr Aufteilungen anschließen. Ein wesentlicher Punkt hierbei ist die genaue Bemessung der Menge der zugegebenen Oligonukleotide. Idealerweise wird zu jeder Reihe von Amplifikationszyklen eine solche Menge der Oligonukleotide hinzugegeben, daß diese völlig oder fast völlig während der Reaktion aufgebraucht werden. Dann kann die Reaktionsmischung jedes Zyklus direkt und automatisch in weitere Schritte übertragen werden.

Die prinzipiell alternative Variante bedarf keiner vorherigen Ligation von Adaptoren an vorgeschnittene DNA. Im Stand der Technik sind einige Verfahren beschrieben, welche genomweite Amplifikationen von DNA mit mehr oder weniger Erfolg erreichen. Alle diese Verfahren müssen für das erfindungsgemäße Verfahren modifiziert werden. Wir haben die Verwendung von drei verschiedenen Verfahren erprobt. Zunächst, und als bevorzugte Variante verwenden wir eine Modifikation der beschriebenen "DOPE"-Technik. Im Gegensatz zu in der Literatur erwähnten Verfahren verwenden wir zwei oder mehr unterschiedliche Oligonukleotide in jeder Amplifikation, welche sich in zwei Klassen einteilen lassen. Diese Klassen sind dadurch charakterisiert, daß in der einen die Base Guanin, in der anderen die Base Cytosin nicht, kaum oder nur im 5'-Bereich vertreten sind. Wenn diese Basen überhaupt in der Sequenz dieser Oligonukleotide vorhanden sind, so dann normalerweise im Kontext der Sequenz 5'-CpG-3'. Dies hat den Zweck, daß diese Klassen von Oligonukleotiden je nur entweder auf den beiden nach der Bisulphit-Behandlung vorliegenden (G-reichen) Strängen, beziehungsweise den mittels Polymerase-Reaktion von diesen Strängen kopierten (C-reichen) Gegensträngen hybridisiert. Durch Kombination von Vertretern dieser beiden Sequenzklassen kann daher eine Amplifikation von Bisulphit-behandelter DNA erreicht werden. Cytosine außerhalb der Sequenz 5'-CpG-3' sollten in der Templat-DNA in den allermeisten Fällen zu Uracil umgewandelt worden sein, so daß für ein effizientes Amplifizieren keine Guanine in dem Oligonukleotid benötigt werden, welches an den Bisulphit-behandelten Strang hybridisiert. Auf dem Gegenstrang gilt dies analog für Guanin. Liegen in diesen Klassen von Oligonukleotiden Guanin beziehungsweise Cytosin im Kontext 5'-CpG-3' vor, so führt dies dazu, daß diese Oligonukleotide auch an potentiell methylierten Positionen hybridisieren können. Dies ist für das vorgeschlagene Verfahren eigentlich nicht von Nutzen. Es kann aber sein, daß die Nachteile so gering sind, daß wesentliche Verfahrensbestandteile auch auf diese Art durchführbar sind. Der Schutzbereich sollte daher auch solche Oligonukleotide einschließen. Genauso ist es denkbar, obwohl im Prinzip eher schädlich für die effiziente Durchführung des Verfahrens, daß einzelne Guanine in Positionen außerhalb des Kontext 5'-CpG-3' vorhanden sind. Normalerweise führt dies bei der für die Amplifikation notwendigen Hybridisierung des Oligonukleotids mit der Ziel-DNA zu nicht-basengepaarten Positionen, was in den meisten Fällen die Effizienz der Amplifikation verschlechtert, also nicht wünschenswert ist. Trotzdem ist die Amplifikation mit Oligonukleotiden, welche eine oder wenige Guanin-Basen enthalten von diesem Strang möglich, wenn auch nicht ideal. Da eine solche Amplifikation immer noch das Wesen der Erfindung erfüllen könnte, so soll auch die Verwendung solcher Oligonukleotide in den Schutzbereich fallen, welche durch Verwendung einiger Guanine nicht strikt in diese Klasse fallen. Besonders die zweite von uns verwendete Technik macht Ausnahmen dieser Art notwendig. Bei dieser werden Oligonukleotide verwendet, welche im Prinzip in deren 3'-Bereich in eine der beschriebenen Sequenzklassen fallen. Im 5'-Bereich dieser Oligonukleotide wird allerdings ein sogenannter "Sequenz-tag" angebracht, welcher in nachfolgenden Schritten zur weiteren Amplifikation verwendet wird. In dieser Variante werden in den ersten Zyklen der Amplifikation die prinzipiell in eine der Klassen fallenden 3'-Bereiche der Oligonukleotide verwendet, um ein großes Spektrum von Fragmenten zu amplifizieren. In nachfolgenden Schritten hat jedes bis dahin amplifizierte Fragment am 3'-Ende eine Sequenz, die den Sequenz-tags entspricht. Diese können nun, analog zur Amplifikation mittels zu Adaptoren komplementären Oligonukleotiden als Hybridisierungspartner für ein Oligonukleotid verwendet werden, welches zur weiteren Amplifikation eingesetzt wird. Das Sequenz-tag dieser ersten Oligonukleotide kann natürlich in zur im 3'-Bereich zur ersten Klasse gehörenden Oligonukleotiden 5'-Bereich Guanin, bei solchen der zweiten Klasse im 5'-Bereich Cytosin enthalten.

Oligonukleotide oder Oligonukleotide, die durch ihre 3'-Bereiche zu einer der beiden Klassen gehören, können unterschiedlich konstruiert sein. Unsere Variante des DOPE-Verfahren benutzt eine Kombination von Oligonukleotiden der beiden Sequenzklassen, die im 3'-Bereich eine determinierte Basensequenz aufweisen. Diese kann innerhalb des erfindungsgemäßen Verfahrens zwischen zwei und zwanzig Basen lang sein. Vor dieser Sequenz liegt ein meist zwischen 5 und 20 Basen langer Abschnitt von "H"-Positionen in der ersten Klasse und "D"-Positionen in der zweiten. Das heißt, daß an diese Positionen in der Synthese des Oligonukleotids im Falle der Klasse "H" eine der drei Basen A, C oder T beziehungsweise im Falle der Klasse "D" eine der Basen A, G oder T eingebaut wurden (wobei die oben genannten, nicht das Wesen der Erfindung betreffenden Ausnahmen in das Schutzrecht eingeschlossen sein sollten). Vor diesem Abschnitt (5') kann (muß aber nicht) ein weiterer Abschnitt mit spezifischer Sequenz liegen. Werden diese Oligonukleotide unter den entsprechenden Bedingungen für die Amplifikation von Bisulphit-behandelter DNA verwendet, so läßt sich reproduzierbar eine über die spezifischen Bereiche der Oligonukleotide definierbare Fraktion des gesamten Genoms amplifizieren. Im Falle der Verwendung von Sequenz-tags kann der 5'-Bereich der Oligonukleotide eine definierte Sequenz aufweisen, welche die Definition der beiden Sequenzklassen durchbricht. Auch sollte Oligonukleotide zwecks Verwendung im Gesamtverfahren im Schutzbereich enthalten sein, welche die Bereiche "H" und "D" im 3'-Bereich enthalten oder in denen die Positionen von definierten Basen mit solchen der Klassen "H" oder "D" in irgendeiner Form alternieren.

Weiterhin sollen unter den Schutzbereich auch solche als Amplifikationsprimer benutzte Oligonukleotide fallen, die innerhalb des Gesamtkonzeptes des Verfahrens benutzt werden und die an ihrem 5'-Terminus sogenannte "hairpin"-Strukturen bilden, solche Moleküle, die das zu dem in der obigen Beschreibung impliziten Basenpaarungsverhalten analoge Basenpaarungsverhalten aufweisen, wie z.B. PNA ("Protein-Nucleic-Acid") basierende Oligonukleotide, chemisch modifizierte Oligonukleotide und solche modifizierten und unmodifizierten Oligonukleotide, die mit anderen als den natürlichen Nukleotiden synthetisiert wurden.

### 4.4. Detektion des Methylierungsstatus von CpG Dinukleotiden

### 4.4.1. Detektion von methylierten CpG Dinukleotiden auf DNA-Chips

In seiner endgültigen Form wird das erfindungsgemäße Verfahren möglicherweise auf der Benutzung eines DNA-Chips beruhen. Die Benutzung eines DNA-Chips stellt daher eine bevorzugte Verfahrensvariante dar. Im Prinzip sind bis zur Amplifikation der Bisulphit-behandelten DNA alle beschriebenen Verfahrensvarianten möglich. Ein Chip zur Durchführung des Verfahrens hat in der bevorzugten Variante die folgende Form: Auf einer dafür vorgesehenen Oberfläche werden nach an sich bekannter Art und Weise mindestens eintausend, in der Regel aber mehr als hunderttausend Oligonukleotide in situ synthetisiert oder mit einer Mikro- oder Nanopipette, einer Stempelähnlichen Apparatur oder über ein Mikro-fluidic-network aufgetragen. Jedes Oligonukleotid ist für eine CpG Position spezifisch, das heißt, das es entweder nur dann mit der Ziel-DNA hybridisiert, wenn die in dem Oligonukleotid enthaltende CpG Position methyliert ist oder nur dann, wenn diese Position eben unmethyliert ist. Für jede Position können daher mindestes (siehe unten) zwei Oligonukleotide aufgebracht werden. Die Zahl der verschiedenen Oligonukleotide ist nach oben hin unbegrenzt und kann sogar das Achtfache aller im Genom enthaltenden CpG Dinukleotide überschreiten. Für jeden Punkt des DNA-Chips ist genau bekannt, welche Oligonukleotid-Sequenz sich an diesem befindet.

Das erfindungsgemäße Verfahren führt eine wesentliche Veränderung in die normale Belegung eines solchen DNA-Chips ein. Auf einem DNA-Chip nach Stand der Technik befinden sich Oligonukleotide, welche mit genomischen oder exprimierten Sequenzen komplementär sind. Das heißt, daß alle Oligonukleotide im Durchschnitt der Basenkomposition der genomischen DNA oder der der exprimierten Sequenzen eines Organismus entsprechen. Die allermeisten Oligonukleotide auf einem solchen DNA-Chip enthalten daher alle vier Basen, im Schnitt entspricht der Anteil der Basen Guanin und Cytosin dem der genomischen und/oder exprimierten Sequenzen.

Anders im Rahmen des erfindungsgemäßen Verfahrens. Im Prinzip können für jede von Oligonukleotiden abgedeckte Sequenz acht Klassen von Oligonukleotiden synthetisiert weklen. Durch die Bisulphit-Behandlung wird die DNA in der Art modifiziert, daß die ursprünglich komplementären oberen und unteren Stränge (Watson bzw. Crick Stränge, auch kodierender und Templat-Strang genannt) nun nicht mehr komplementär sind. Das heißt, daß Oligonukleotide für beide Stränge synthetisiert werden können. Dies bietet sich an, da die beiden Stränge sich so als interne Kontrollen füreinander benutzen lassen. Die Hybridisierungsverhalten der beiden unterschiedlichen Stränge mit den jeweils auf sie passenden Oligonukleotiden ist auf Grund der teilweise drastischen Sequenzunterschiede unterschiedlich. Dies führt dazu, daß, wenn auf beiden Strängen dasselbe Ergebnis erreicht wird, dieses als unabhängig bestätigt angesehen werden kann. Auch soll quantifiziert werden, wie hoch an jeder zu testenden Position die Anteile von Methyl-Cytosin und Cytosin sind. Die Verwendung beider Stränge erlaubt durch die Bewertung unterschiedlicher Hybridisierungsereignisse für jede individuelle CpG-Position eine von den unterschiedlichen Hybridisierung-Parametern der Oligonukleotide unabhängige Quantifizierung der Daten. Hintergrundfehler werden so minimiert.

Nach der Bisulphit-Behandlung sind nicht nur beide Stränge unterschiedlich. Nach der Behandlung wird ja in jedem Fall eine Amplifikation durchgeführt, welche an jedem der beiden Stränge wiederum die Neu-Synthese eines komplementären Gegenstranges beinhaltet. Genauso wenig, wie die ursprünglichen Stränge nach einer Bisulphit-Behandlung miteinander komplementär sind, sind auch die beiden Gegenstränge nicht miteinander komplementär. Auch ein während einer Amplifikation neu synthetisierter Gegenstrang ist nicht mit dem ursprünglichen anderen (dem, an welchem der Gegenstrang nicht synthetisiert wurde) Strang komplementär. Es entstehen also vier verschiedene Hybridisierungsziele für jede ursprüngliche CpG Position. Alle diese vier Stränge enthalten (wir gehen hier von symmetrischer, das heißt Methylierung an beiden Strängen einer CpG Position aus) die gleiche Information, hybridisieren aber mit Oligonukleotiden unterschiedlicher Sequenz. Also wird auf diese Weise jede über eine beliebige CpG Position erhaltene Information vierfach unabhängig belegt. Trotzdem ist die Signalstärke für die vier verschiedenen Oligonukleotide nicht direkt (außer durch Erfahrungswerte, welche beim Gebrauch des Systems geschaffen werden) mit dem Grad der Methylierung einer Position korrelierbar. Es ist nämlich so, daß unterschiedliche Fragmente in einer enzymatischen Amplifikation auch unterschiedlich effizient amplifiziert werden, die Stärke eines Signals also nicht unbedingt mit dem Grad der Methylierung, sondern auch mit der Effizienz der Amplifikation des die CpG Position enthaltenden Fragments korreliert. Es müssen daher in jedem Fall für alle vier Stränge beide möglichen Oligonukleotide analysiert werden, zum einen jenes, welches nur dann hybridisiert, wenn die zu untersuchende CpG Position methyliert ist (also CpG enthält), zum anderen jenes, welches nur im Falle einer unmethylierten CpG Position hybridisiert (also kein CpG enthält). Die beiden möglichen Varianten eines DNA-Stranges, nämlich die methylierte und unmethylierte Variante werden mit weitgehend identischer Effizienz amplifiziert, lassen also einen Vergleich zu. Da nun für alle vier Stränge diese komplementäre Information zur Verfügung steht, lassen sich auch alle vier Stränge dazu verwenden, das Gesamtergebnis abzusichern. Im Rahmen des erfindungsgemäßen Verfahrens sind die hauptsächlichen Kriterien, welche die Oligonukleotide gegenüber anderen Verfahren abgrenzen, daß sie eben nur jeweils drei der vier Basen enthalten. Die Oligonukleotide, welche zu den ursprünglichen DNA-Strängen komplementär sind, enthalten nur die Base C, nicht aber die Base G. Nur in der Hälfte aller dieser Oligonukleotide befindet sich genau ein Guanin, nämlich im Kontext CpG genau an der Stelle, welche auf ihren Methylierungszustand getestet werden soll. Die zweite Klasse der Oligonukleotide, welche zum in der Amplifikation generierten Gegenstrang der ursprünglichen DNA komplementär ist, enthält im Gegensatz hierzu die Base Cytosin nur an solchen Stellen, für die der Methylierungszustand getestet werden soll. Diejenigen Oligonukleotide, welche nur dann mit der Ziel-DNA hybridisieren, wenn die von ihnen getestete Position unmethyliert vorliegt, enthalten (je nach Strang) entweder gar kein Cytosin oder gar kein Guanin. Innerhalb des vorgeschlagenen Verfahrens sind die beschriebenen acht Klassen von Oligonukleotiden natürlich auch noch in anderer Hinsicht variabel. Auch können mehrere Vertreter einer Klasse gleichzeitig für die Untersuchung jeder individuellen methylierbaren Position eingesetzt werden. Es ist zum Beispiel nicht in allen Fällen offensichtlich, wie viele Basen auf jeder Seite der potentiellen Methyl-Position auf jeder Seite in das Oligonukleotid eingeschlossen werden. Die methylierbare Position muß nicht genau in der Mitte des Oligonukleotids liegen. Daher sind für jede zu testende Position viele Permutationen möglich.

In den Extremfällen liegt die zu testende Position an einem der Extreme des Oligonukleotids oder (allerdings schon Bestandteil einer weiteren Verfahrensvariante) sogar eine Position hinter dem 3'-Terminus, so daß die Präsenz von Cytosin oder Guanin (mithin von Methylierung der ursprünglichen Probe) nicht durch einfache Hybridisierung, sondern durch den Nachweis einer Primer-Extension nachgewiesen wird. In dieser Verfahrensvariante werden modifizierte Nukleotid-Triphosphate (in der Art, daß zwar der Einbau eines solchen Nukleotids an das 3'-Ende eines Primers möglich ist, aber keine weitere Verlängerung über dieses Oligonukleotid hinaus. In der Regel werden hier 2',3'-Dideoxy-Analoge der vier Nukleotid-Triphosphate verwendet) mit einer für jedes der vier Nukleotide unterschiedlichen Markierung zu der Ziel DNA gegeben. welche dann auf dem Chip mit den Oligonukleotiden hybridisiert wird. Anstatt nun die Hybridisierung direkt nachzuweisen, wird eine Polymerase hinzugegeben und an jeder Position genau ein Nukleotid an das 3'-Ende der Oligonukleotids synthetisiert. Das zu dem am 3'-Ende des Oligonukleotids eingebauten Nukleotid komplementäre Nukleotid entspricht genau dem, welches auf der mit dem Oligonukleotid hybridisierten Ziel-DNA eine Position 5' vor dem Oligonukleotid liegt. In unserem Verfahren ist diese Position eine in der ursprünglichen DNA methylierbar. Wenn nun also (je nach Strang) die Position in der DNA-Probe methyliert war, so befindet sich an dieser Position ein C; an das Oligonukleotid wird also ein G "angebaut". Sind die dGTPs (oder Analoge dieses Nukleotids) nun eindeutig markiert und, (was ja Voraussetzung ist) die Oligonukleotidsequenzen an allen Positionen bekannt, so kann in diesem Falle der Nachweis des Einbaus von Guanin dazu dienen, die Präsenz eine Methylgruppe in der ursprünglichen Probe nachzuweisen. Wird an das gleiche Oligonukleotid ein Adenin angehängt, so ist der Nachweis von Thymidin gelungen, mithin der Nachweis, daß die untersuchte Position unmethyliert vorlag. Der gleiche Nachweis, nur mit den markierten ddNTPs Cytosin und Thymidin kann auf den in der Amplifikation hergestellten Gegensträngen erfolgen. In dieser Verfahrensvariante enthalten die Oligonukleotide der beiden Sequenzklassen je entweder kein Cytosin oder kein Guanin. Trotzdem kann in Ausnahmefällen von dieser Regel abgesehen werden (zum Beispiel, wenn eine Position als immer methyliert oder immer unmethyliert bekannt ist oder der Methylierungszustand der Position auf das Hybridisierungsverhalten des Oligonukleotids keinen Einfluß hat. Weiterhin kann auch eine oder wenige "Mismatch-Position" innerhalb der Oligonukleotide möglicherweise trotz deren im Prinzip schädlicher Wirkung wesentliche Ansprüche des Verfahrens erfüllen. Solche nicht strikt in die Sequenzklassen fallenden Oligonukleotide, welche wesentliche Verfahrensbestandteile erfüllen, sollten daher in den Patentschutz eingeschlossen sein. Außerdem kann die Befestigung der Oligonukleotide auf der Oberfläche des DNA-Chips über Sequenz-tags an den Oligonukleotiden erfolgen, die gegen eine generische Sequenz von auf der Oberfläche befestigten Oligonukleotiden komplementär sind. Solche Oligonukleotide fallen nur in den für die Hvbridisierung mit der DNA-Probe zur Verfügung stehenden Bereichen in die definierten Sequenzklassen). Weiter sollen unter den Schutzbereich auch solche als Hybridisierungspartner auf der Oberfläche von DNA-Chips benutzte Oligonukleotide fallen, die das zu dem in der obigen Beschreibung impliziten Basenpaarungsverhalten analoge Basenpaarungsverhalten aufweisen, wie z.B. PNA ("Protein-Nucleic-Acid") basierte Oligonukleotide, chemisch modifizierte Oligonukleotide und solche modifizierten und unmodifizierten Oligonukleotide, die mit anderen als den natürlichen Nukleotiden synthetisiert wurden.

Dies gilt natürlich für alle Verfahrensvarianten, welche auf Hybridisierung von Oligonukleotiden direkt mit der zu testenden Position oder auf Primer-Extension durch nur eine Base beruhen: In der Regel wird nur eine Position getestet, und diese Position macht auch den gesamten Cytosin- bzw. Guanin-Gehalt eines Oligonukleotids aus. Dennoch können Ausnahmen von dieser Regel in Einzelfällen belanglos sein und sind daher gleichfalls Gegenstand der vorliegenden Erfindung.

Die Detektion der verschieden markierten Nukleotid-Analoge in einer Primer-Extensions-Reaktion auf einem (wie auch immer gearteten) DNA-Chip läßt sich auf verschiedenste Art bewerkstelligen. Eine bevorzugte Variante ist die Detektion auf an sich bekannte Art mit einer CCD-Camera, welche Fluoreszenzsignale registriert, welche auf dem Chip die erfolgte Bindung eines (natürlich fluoreszenz-markierten) Nukleotids anzeigen. Dabei ist in der oben beschriebenen Verfahrensvariante jedes der Nukleotid-Analoge mit einer unterschiedlichen Farbe markiert, so daß an jeder Position nachgewiesen werden kann, welches Nukleotid eingebaut wurde.

Es ist aber auch eine wichtige Variante, jedes der vier Nukleotid-Analoge mit einem chemischen Molekül zu markieren, welches daraufhin durch Beschuß mit dem Laser eines MALDI-TOF von Nukleotid photochemisch (oder durch die generierte Hitze oder einen analogen Prozeß) abgetrennt wird und danach direkt ionisiert auf sein Molekulargewicht hin analysiert wird. Der Laser des MALDI-TOF Gerätes kann hierbei jede Position des Chips genau ansteuern, also auch für jede Position auf dem Chip getrennt ermitteln, welche Massemodifikation sich an der entsprechenden Stelle befand. Oft (da ja methylierte und unmethylierte Ziel-DNA in dieser Variante mit den gleichen Oligonukleotiden hybridisieren und der Methylierungszustand durch die Markierung des eingebauten Nukleotids ermittelt wird), werden an jeder Position zwei Markierungen detektiert (gilt natürlich auch für Fluoreszenz-Markierungen) und die beiden Signale müssen quantifiziert und miteinander verglichen werden, um den Methylierungsgrad zu ermitteln.

In der zur Zeit bevorzugten Verfahrensvariante wird allerdings die Detektion mittels Fluoreszenz benutzt. Weiterhin werden Hybridisierungen direkt detektiert und keine Primer-Extensions-Reaktion durchgeführt.

### 4.4.2. Nachweise des Methylierungsstatus von Cytosin durch massenspektrometrische Längenmessung von "Primer-Extension" Produkten

Es wurde eine Verfahrensvariante entwickelt, welche die Detektion von extremen Zahlen von Cytosinen und/oder Guaninen in Bisulphit-behandelter DNA mittels massenspektrometrischer Längenmessung in MALDI basierten Massenspektrometern erlaubt. Die Grundlage dieser Technologie, welche für dieses Verfahren modifiziert wurde, ist oben beschrieben.

Wir benutzten im vorgeschlagenen Verfahren solche Oligonukleotide, die durch ihre Zugehörigkeit zu einer der beiden schon oben definierten Sequenzklassen mit größter Wahrscheinlichkeit nur auf einem der beiden Stränge Bisulphit-behandelter DNA hybridisieren. Oligonukleotide, welche in dieser Verfahrensvariante verwendet werden, können die Detektion von Cytosin und/oder Guanin aus Amplifikationsgemischen aller oben beschriebener Methoden der Amplifikation erreichen. Das heißt, daß im Prinzip sowohl solche Oligonukleotide verwendet werden können, die mit vor der Bisulphit-Behandlung an die Fragmente der Probe ligierten Adaptoren komplementär sind, als auch solche, die an nicht definierten Positionen auf den, auf die anderen Arten amplifizierten Fragmenten, hybridisieren.

Die bevorzugten Verfahrensvarianten beinhalten die Verwendung von DNA-Proben, an deren Restriktionsfragmente Adaptoren ligiert wurden (und dann nach der Bisulphit-Behandlung amplifiziert werden), oder solche, die mit Oligonukleotiden amplifiziert wurden, welche in deren 5'-Bereich konstante Sequenz-tags enthalten. Die Adaptoren werden zu diesem Zweck so synthetisiert, daß nach einer Bisulphit-Behandlung die beiden Stränge, das heißt der originale, Bisulphit-modifizierte und der während der Amplifikation neu-synthetisierte Strang in Bezug auf Cytosin beziehungsweise Guanin Gehalt derart unterschiedlich sind, daß Oligonukleotide für eine Primer-Extensions-Reaktion hergestellt werden können, die einen der beiden Stränge spezifisch erkennen. Das heißt, daß auch in diesem Fall zwei Sequenz-Klassen von Oligonukleotiden unterschieden werden können. Die verwendeten Oligonukleotide haben die Eigenschaft, daß ihr 3'-Bereich über die bekannte Adaptor-Sequenz und über die von der Restriktionsendonuklease erkannte und daher bekannte Sequenz hinaus in den unbekannten Bereich der Proben-DNA hineinreichen. In dem Fall, daß schon die generische Amplifikation wie oben beschrieben mit schrittweise längeren Oligonukleotiden in seriell unterteilten, separaten Reaktionen durchgeführt wurden, so reichen die hier definierten Oligonukleotide auch über diesen bekannten Bereich hinaus. Dabei können die Oligonukleotide zwischen zwei und 20 Basen in den unbekannten Bereich hineinreichen. Die Mischung der Fragmente aus der ersten oder den ersten, generischen Amplifikationen wird nun unterteilt und mit für jede (Sub-)Reaktion unterschiedlichen Oligonukleotiden gemischt. In jeder Subreaktion ist dabei bekannt, welche Oligonukleotidsequenz hinzugegeben wird, die Subreaktionen unterscheiden sich nur durch die Sequenz der hinzugegebenen Oligonukleotide. Es ist dabei nicht wesentlich, ob die Sequenz der Oligonukleotide genau definiert ist, oder einzelne Positionen mit den oben definierten, degenerierten Nukleotid-Positionen "H" oder "D" besetzt sind. Die Verwendung von degenerierten Positionen erlaubt eine Verwendung von längeren Bereichen, die in den unbekannten Bereich hineinreichen und damit eventuell eine genauere Regulation und Abstufung der Zahl und Art der in einer solchen Reaktion generierten Extensions-Fragmente.

Mit allen unterschiedlichen Subreaktionen wird eine Polymerase-Reaktion mit den folgenden Komponenten durchgeführt. Diejenigen Reaktionen, welche solche Oligonukleotide enthalten, die mit einem Cytosin-armen Strang (entsprechend den Originalsträngen der Bisulphit-behandelten DNA) hybridisieren, enthalten die Nukleotide dATP, dCTP, dTTP und einen dem Basenpaarungsverhalten nach dem Nukleotid dGTP analogen Terminator, wie zum Beispiel ddGTP oder ein funktionell äquivalentes Nukleotid. Die Reaktionen mit Oligonukleotiden der anderen Sequenzklasse enthalten ein Gemisch bestehend aus dATP. dGTP, dTTP und einen dem Basenpaarungsverhalten nach dem Nukleotid dGTP analogen Terminator, wie zum Beispiel ddCTP oder ein funktionell äquivalentes Nukleotid. Eine Polymerase-Reaktion wird nun von den Oligonukleotiden ausgehend auf dem einen (Cytsosin-armen) Strang nur bis zum ersten Cytosin beziehungsweise auf dem anderen Strang bis zum ersten Guanin, einen neuen DNA-Strang synthetisieren.

Es ist für eine massenspektrometrische Analyse auch sinnvoll, anstatt der natürlich vorkommenden Nukleotide solche zu benutzen, die in bekannter Art und Weise der Art chemisch modifiziert sind, daß die nachfolgende massenspektrometrische Analyse der Extensions-Produkte erleichtert wird. In unserer Variante werden hierzu Phosphothioat-Analoge der natürlichen Nukleotide benutzt. Diese können in einem nachfolgenden Schritt alkyliert werden, was die Rückrad-Ladungen der DNA eliminiert und die Analysequalität und Sensitivität erhöht. Aber auch andere Modifikationen sollen in den Schutzbereich fallen, welche dieses Ziel verfolgen. Weiterhin kann die Modifizierung der Ladung der verwendeten Oligonukleotide auch deren Hybridisierungseigenschaften verbessern oder verändern.

Ziel dieser Verfahrensvariante ist die Herstellung von Fragment-Populationen in den einzelnen Reaktionen, die so, und nur so komplex sind, daß diese separat auf einer Gelelektrophorese oder eben durch massenspektrometrische Analyse der Länge nach aufgetrennt werden können. Dadurch ist es nötig, die Zahl der synthetisierten Fragmente über die Länge des in den unbekannten Sequenz-Bereich hineinragenden Teil der Oligonukleotide und den Grad der Degeneriertheit so einzustellen, daß diese pro Reaktion zwischen einem und möglicherweise bis zu einigen Tausend verschiedenen Fragmenten liegt.

Die einzelnen Reaktionen werden nun in der bevorzugten Variante separat auf definierte Koordinaten der Ionenquelle eines Massenspektrometers aufgetragen. Die massenspektrometrische Analyse ermittelt dann für die individuellen Koordinaten die Fragmentspektren. Bei bis zu einigen tausend Koordinaten auf der Ionenquelle eines Massenspektrometers und einigen hundert Fragmenten pro Spektrum, von denen jedes eine Cytosin oder Guanin Position als Indikator der Methylierung beurteilt, können also bis zu einigen Hunderttausend einzelne CpG Dinukleotide beurteilt werden.

Analog kann auch die Detektion von Fragmentspektren erfolgen, die von einer Fragmentpopulation generiert wurden, die ohne die Ligation von Adaptoren mittels der oben beschriebenen Oligonukleotid-Primer amplifiziert wurde. Bei dieser Variante wird auf die zu den Adaptoren komplementäre Sequenz verzichtet und statt dessen ein mehrere degenerierte Positionen enthaltender 5'-Bereich verwendet.

In dem Fall, daß die Bisulphit-behandelte DNA mit solchen Oligonukleotiden voramplifiziert wurde, die in ihrem 3'-Bereich die oben beschriebenen (5'-)Sequenz-tags enthalten, können diese auch analog zur den Adaptorsequenzen als konstanter Bereich für eine Hybridisierung mit Oligonukleotiden benutzt werden, wie sie in diesem Abschnitt oben beschrieben wurden.

### 4.4.4. Detektion des Methylierungsstatus von Cytosin durch massenspektrometrischen Nachweis chemisch modifizierter Oligonukleotide

Eine weitere Verfahrensvariante macht sich ein an sich bekanntes Verfahren zunutze, das die massenspektrometrische Identifikation von bestimmten Sequenzen indirekt über den Nachweis von an ein Oligonukleotid angebrachten chemischen Modifikationen ermöglicht.

In den oben beschriebenen massenspektrometrischen Detektionsvarianten werden viele verschiedene Primer-Extensions-Reaktionen mit jeweils einer oder wenigen Oligonukleotid-Sequenzen durchgeführt. Im Prinzip wird dann die extreme Anzahl verschiedener analysierbarer Fragmente nur durch das Aufteilen in viele verschiedene Reaktionen (und Koordinaten auf einer MALDI Ionenquelle) erreicht.

Wird jede verschiedene Primer-Sequenz mit einer chemischen Modifikation versehen, so kann - bei Verwendung einer anderen Analysetechnik als nur MALDI allein - auf dieses Auftrennen verzichtet werden.

Praktisch bedeutet dies, daß alle verschiedenen verwendeten Primer schon in der Synthese oder nachträglich mit einer solchen Chemie versehen werden, die im Prinzip zwei Anforderungen erfüllt. Zum einen darf die Längenauftrennung der generierten Fragmente nicht verhindert werden. Zum zweiten muß die Art der Modifikationen erlauben diese im zweiten, sich an die Kapillarelektrophorese anschließenden Analyse-Schritt zu unterscheiden. Damit ist die Art der Modifikationen von der Art der Analyse im zweiten Schritt abhängig. In der bevorzugten Verfahrensvariante sind die 5'-Enden der Primer mit kurzen Peptidsequenzen versehen, die in einem nachfolgenden Schritt mit vielen gebräuchlichen Analyse-Verfahren aufgetrennt werden können. Einer der großen Vorteile einer solchen Variante ist, daß auch im ersten, unspezifischen Amplifikationsschritt eine wesentlich kleinere Gesamtmenge von DNA amplifiziert werden muß, da diese Menge ja nicht noch auf weitere Reaktionen aufgeteilt werden muß. Die zweite Dimension der Trennung, welche in den oben beschriebenen Varianten durch das Auftrennen in einzelne Reaktionen erreicht wird, kann in der bevorzugten Verfahrensvariante dieser Ausführung des erfindungsgemäßen Verfahrens dadurch erreicht werden, daß die Trennung der generierten Fragmente zunächst durch eine Kapillarelektrophorese geschieht. Dabei ist es für ein korrektes Ergebnis unwesentlich, ob die chemischen Modifizierungen an den Fragmenten das Laufverhalten der Fragmente beeinflussen oder nicht, wenn nur eine Trennung nach der Länge möglich bleibt. In jeder "Fraktion", die das Ende der Kapillarelektrophorese erreicht, befinden sich viele Fragmente gleichen elektrophoretischen Laufverhaltens, die sich nur durch die chemische Modifikation in ihren jeweiligen 5'-Bereich (im Bereich des Primers, welcher für die Extensions-Reaktion eingesetzt wurde) unterscheiden. Diese, nach ihrem elektrophoretischen Laufverhalten aufgetrennten Fragment-Populationen, werden nun in einem zweiten Schritt auf die chemischen Modifikationen hin untersucht. Die bevorzugte Verfahrensvariante ist dabei die direkte Einspritzung des Austrittsvolumens der Kapillarelektrophorese in ein Fast-Atom-Bombardement (FAB-MS), Electron-Spray (ESI-MS), Auftragen auf ein MALDI Massenspektrometer oder eine äquivalente Analyseapparatur.

Konkret wird eine solche Variante zum Beispiel wie folgt ausgeführt. DNA wird wie beschrieben aus Zellen präpariert, mit einer Restriktionsendonuklease vorgeschnitten, mit Adaptoren versehen und durch eine temperierbare, für kleine Moleküle poröse Kapillare geführt, in welcher die Reaktionsschritte der Bisulphit-Reaktion durch Zu- und Abfuhr der Reagenzien durch Dialyse ausgeführt werden. Das Volumen der Gesamtreaktion ist dabei sehr gering. Nach erfolgter Bisulphit-Reaktion können der Kapillare durch Kreuzungen mit weiteren, zuführenden Kapillaren die für eine Amplifikation notwendigen Reagenzien zugeführt werden und die Amplifikation dann in derselben beheizbaren Kapillare durchgeführt werden. Es ist aber auch möglich die Amplifikation nicht direkt in der Kapillare, sondern in einem an diese Kapillare angeschlossenen Behälter durchzuführen. An die generische Amplifikation der genomischen Fragmente schließt sich ein zweiter, wie beschrieben linearer Verlängerungsschritt an, welcher mit einem Gemisch von chemisch modifizierten und dadurch ihrer Masse nach unterscheidbaren Oligonukleotiden durchgeführt wird, welche zu den Bisulphit-modifizierten Adaptoren komplementär sind. Es folgt eine Längentrennung der Verlängerungsprodukte in einem weiteren Abschnitt der Kapillare und gegebenenfalls eine weitere Dialyse gegen einen mit einer massenspektrometrischen Analyse kompatiblen Puffer, wie zum Beispiel Ammonium-Sulphat.

Jede einzelne Fraktion wird auf eine Koordinate der Ionenquelle eines Massenspektrometers aufgetragen und dann jede Koordinate auf die Präsenz der chemischen Modifikationen untersucht, welche sich durch ihre Masse unterscheiden. Bei dieser Variante wird aus apparativen Gründen bevorzugt ein MALDI-TOF benutzt, welches über eine sehr große Ionenquelle verfügt, wodurch sehr viele verschiedene Koordinaten in kurzer Abfolge analysiert werden können.

Weitere Verfahrensvarianten ergeben sich aus der Tatsache, daß das beschriebene Verfahren ganz allgemein alle Meßpunkte auf zwei Dimensionen generiert, eine Notwendigkeit bei der Erstellung von Zahlen von Meßpunkten, wie hier beschrieben. Auf DNA Chips sind diese zwei Dimensionen räumlich angeordnet, ebenso bei der beschriebenen Variante der Analyse einzelner "Subreaktionen" auf der Ionenquelle eines MALDI-TOFs. Bei der kapillarelektrophoretischen Variante werden die zwei Dimensionen durch Hintereinanderschalten zweier nach unterschiedlichen Kriterien separierenden Trennmethoden erreicht. Es gibt noch mehrere Varianten eines solchen Verfahrens, welche, da diese dem erfinderischen Gesamtkonzept entsprechen, mit unter den Patentschutz fallen sollen. Es ist für die Messung an sehr vielen Punkten im Rahmen des erfindungsgemäßen Verfahrens nicht unbedingt notwendig, über jeden Meßpunkt zu wissen, welchen Ursprungs dieser Meßpunkt ist. Für viele Anwendungen des Verfahrens ist es ausreichend, eine riesige Menge abstrakter Daten mit phänotypischen Eigenschaften von Zellen zu korrelieren. Daher ergibt sich ein wesentlich größeres Spektrum an möglichen Analyse-Verfahren. In der Regel ist allerdings eine Kapillarelektrophorese in allen Varianten, in denen nicht direkt ein erfolgtes Hybridisierungsereignis nachgewiesen wird (in sich eine Analysedimension), notwendig.

### 4.5. Analyse der generierten Daten

Die hauptsächlichen Ansprüche beziehen sich auf das Verfahren im allgemeinen, solche komplexen Methylierungs-Fingerabdrücke herzustellen und mittels eines Auswertealgorithmus mit phänotypischen Charakteristika der untersuchten Zellen zu korrelieren. Der Patentschutz soll sich aber, da die Generierung und Verwendung der Daten in Kombination die eigentliche erfinderische Höhe aufweist, auf alle Verfahren beziehen, welche zur Beschaffung von Methlyierungsdaten mit dem Ziel der erfindungsgemäßen Auswertung dieser Daten geeignet sind.

Am Ende aller der oben beschriebenen Verfahrensschritte steht eine riesige Zahl von Meßpunkten. Drei verschiedene Arten von Werten können dabei entstehen. Reine Plus-Minus Signale für Positionen, welche entweder auf allen analysierten Chromosomen methyliert oder unmethyliert vorliegen, sind wahrscheinlich nicht die zahlenmäßig größte Gruppe der detektierbaren methylierbaren Positionen. Sehr viele Positionen werden solche Signale generieren, welche mit den oben beschriebenen Methoden quantifiziert werden müssen.

Im Prinzip ist die Analyse reiner Plus-Minus Signale wesentlich einfacher. Die Analysestrategie soll wie folgt aussehen. Aus vielen verschiedenen DNA-Proben bekannten Ursprungs (z.B. aus Antikörper-markierten und per Immunofluoreszenz isolierten Zellen gleichen Phänotyps) werden in einer großen Anzahl von Versuchen Daten generiert und deren Reproduzierbarkeit überprüft. Solche Positionen, die nicht reproduzierbare Ergebnisse liefern, werden von allen anderen logisch getrennt, da zunächst nicht beurteilt werden kann, ob solche Unterschiede an einzelnen Positionen biologisch signifikant sind. Diese Testreihen sollen an Zellen verschiedenen Typs durchgeführt werden. Das Ergebnis dieser Testreihen sollte eine große, heute noch unbekannte Zahl von CpG Dinukleotiden sein, die beim Vergleich eines beliebigen Paares von Zelltypen einen reproduzierbaren Unterschied in ihrem Methylierungsstatus ergeben. Nicht alle im direkten Vergleich zweier Zelltypen unterschiedlichen Positionen werden in allen solchen Vergleichen über ihre Unterschiedlichkeit informativ sein. Analysiert man nun alle Positionen, die in mindestens einem Zelltyp-Vergleich unterschiedlich sind, so kann für jeden getesteten Zelltyp ein charakteristisches Muster erstellt werden. Damit kann dann eine DNA-Probe unbekannten Ursprungs einem Zelltyp zugeordnet werden. Diese Muster sind nicht unbedingt an allen getesteten Positionen konstant. Es kann zur Zeit nicht beurteilt werden (das erfindungsgemäße Verfahren schafft ja erst die Grundlage für eine solche Beurteilung), wie stark das Methylierungsmuster eines Zelltyps aus einer individuellen Probe vom charakteristischen Mittel abweicht. Im Idealfall sind die generierten Muster pro Zelltyp und Individuum so konstant, daß ohne großen Aufwand die Identifizierung eines solchen Gewebes erfolgen kann. Eine vorbestimmte Matrize mit definierten charakteristischen Signalkoordinaten kann dann direkt zur Zuordnung der Probe zu einem Zelltyp dienen. Im kompliziertesten Fall ist nicht ein einziges definierbares Muster von Signalen für einen Zelltyp charakteristisch, sondern viele solche Muster, die zwar im Grunde charakteristisch sind, aber nicht offensichtlich als solche identifiziert werden können. Es ist, dies kann aus dem Stand der Technik bei der Methylierungsanalyse abgeleitet werden, nämlich möglich, daß anscheinend sehr unterschiedliche Muster sehr ähnliche Funktionen beinhalten. Zur Zeit ist aber keine Aussage über den Grad dieser Schwierigkeit möglich, da das erfindungsgemäße Verfahren ja erst die Möglichkeit der Beurteilung einer solchen Situation bereitstellt. Es kann also sein, daß mit konventionellen Methoden, sozusagen "per Auge", eine Probe keinem Ursprung zugeordnet werden kann. In diesem Fall beinhaltet das vorgeschlagene Verfahren die Möglichkeit ein sogenanntes "neuronales Netzwerk" (NN) mit den in den Testreihen ermittelten Daten zu "trainieren". In der Praxis sieht das so aus, daß sehr viele Testreihen mit Zell-DNA-Proben gefahren werden und in die Eingabe-Ebene des NN eingespeist werden. Gleichzeitig mit den Methylierungsdaten der Probe wird dem NN die Information über den Ursprung der Proben angeboten. Neuronale Netzwerke können nun nach einer genügenden Zahl von Versuchen gewissermaßen lernen, welche Muster zu welchen Zell-Typen gehören. Auf diese Weise können so extrem komplexe und offensichtlich undurchsichtige Muster klassifiziert werden, die dem menschlichen Verstand und konventionellen Algorithmen völlig chaotisch erscheinen.

Es ist, wie gesagt noch nicht abzusehen, wie komplex und anscheinend chaotisch die generierten Muster erscheinen werden. Jeder Fall zwischen den beschriebenen ist möglich. Daher soll jedes Verfahren, welches sich die Zuordnung von komplexen Methylierungsmustern zu Zelltypen bekannten Ursprungs in Testreihen zunutze macht um sich damit in die Lage zu versetzen Zelltypen unbekannten Ursprungs zu klassifizieren, Gegenstand der vorleigenden Erfindung sein.

Komplizierter wird die Analyse der Daten mit Sicherheit bei der Analyse von Zellen aberranten Ursprungs. Der Zweck des vorgeschlagenen Verfahrens ist es, die Klassifizierung unbekannter, erkrankter Zelltypen zu erlauben. Mit den Methylierungsdaten der untersuchten Proben müssen daher phänotypische Parameter der untersuchten Zellen während der Testreihen dem NN und/oder anderen Auswertesystemen angeboten werden, wobei zunächst einmal nicht klar ist, welche dieser phänotypischen Daten überhaupt mit dem Methylierungsmuster korreliert werden müssen und im Rahmen einer solchen Korrelation sinnvolle Daten ergeben. In diesen Fällen gelten vermehrt die Schwierigkeiten, die aus scheinbar chaotischen aber doch grundsätzlich klassifizierbaren Datenmengen entstehen. Es kann der Fall sein, daß im Falle von entarteten Zellen verschiedene epigenotypische Zustände zu ähnlichen phänotypischen Merkmalen führen. Solche Situationen werden besonders gut von NNs erkannt und können dann zur Definition von neuen, genauer differenzierten Phänotypen führen, was einer der Hauptzwecke des vorgeschlagenen Verfahrens ist. Wünschenswert ist daher die explizite Einbeziehung in den Patentschutz der Verwendung der verschiedenen Arten von neuronalen Netzwerken bei der Analyse von Methylierungsdaten zwecks Korrelation der Methylierungsmuster mit phänotypischen Daten. Aber auch die einfacheren Situationen können das Wesen der Erfindung erfüllen und sollten vom Patentschutz daher nicht ausgenommen sein.

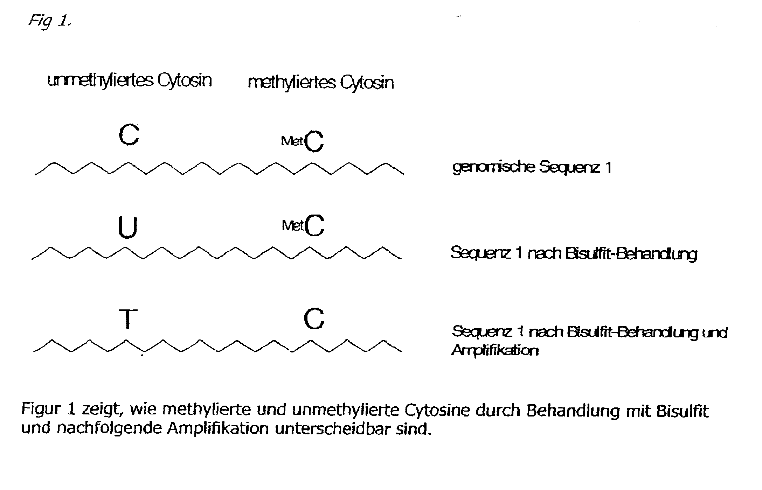

## Patentansprüche

1. Verfahren zur Charakterisierung, Klassifizierung und Unterscheidung von Geweben und Zelltypen, zur Vorhersage des Verhaltens von Geweben und Gruppen von Zellen und zur Identifizierung von in ihrer Expression veränderten Genen, **dadurch gekennzeichnet, daß**
in einer aus einer beliebigen Gewebeprobe gewonnenen unbehandelten, gescherten oder mittels einer Restriktions-Endonuklease gespaltenen genomischen DNA auf an sich bekannte Art und Weise die Base Cytosin, aber nicht 5-Methylcytosin durch Behandlung mit einer Bisulphit-Lösung in Uracil umgewandelt wird
Fraktionen der so behandelten genomischen DNA durch Verwendung von unspezifisch hybridisierenden Oligonukleotiden und/oder einer Mischung aus zwei oder mehr verschiedenen, spezifisch hybridisierenden Oligonukleotiden und/oder von solchen Oligonukleotiden, welche zu - vor der Bisulphit-Behandlung an die Enden der gespaltenen DNA ligierten - Adaptor-Oligonukleotiden komplementär sind, amplifiziert werden,
man insgesamt eine solche Menge der verbleibenden Cytosine auf dem Guanin-reichen DNA-Strang und/oder Guanine auf dem Cytosin-reichen DNA-Strang aus den amplifizierten Fraktionen durch eine Hybridisierung oder Polymerase Reaktion nachweist, daß man die bei einer solchen Analyse generierten und automatisch an einen Verarbeitungsalgorithmus übertragenen Daten analysiert und auf den Phänotyp des analysierten Zellmaterials rückschließt.

2. Verfahren nach Anspruch 1, **dadurch gekennzeichnet, daß**
die aus dieser Analyse gewonnenen Daten mehrerer oder vieler solcher Versuche an DNA-Proben aus phänotypisch gleichen oder ähnlichen Zellen oder Geweben in einer Trainingsphase über ein neuronales Netzwerk oder andere Auswertealgorithmen mit dem Phänotyp der Zellen, deren DNA untersucht wurde, korreliert werden,
die bei dieser Trainingsphase in den Auswertealgorithmus aufgenommenen Daten über den Zusammenhang zwischen Phänotyp und Methylierungsmuster dazu benutzt werden, durch Generierung eines Methylierungsmusters einer DNA-Probe unbekannten Ursprungs den Phänotyp der Zellen, deren DNA untersucht wurde, abzuleiten oder
die bei dieser Trainingsphase in den Auswertealgorithmus aufgenommennen Daten über das Methylierungsmuster der DNA eines bekannten Zelltyps dazu benutzt werden, solche Cytosin-Positionen zu identifizieren, welche in der untersuchten DNA von dem in der Trainingsphase ermittelten Methylierungszustand abweichen.

3. Verfahren nach Anspruch 1, **dadurch gekennzeichnet, daß** die DNA vor der Behandlung mit Bisulphit mit solchen Restriktionsendonukleasen gespalten wird, welche Cytosin im Kontext 5'-CpG-3' in ihrer Erkennungssequenz enthalten und die DNA nur an solchen dieser Erkennungssequenzen spalten, in denen Cytosin im Kontext 5'-CpG-3' an der 5'-Position unmethyliert vorliegt.

4. Verfahren nach Anspruch 1, **dadurch gekennzeichnet, daß**
bevor die genomische DNA auf an sich bekannte Art und Weise mit einer Bisulphit Lösung modifiziert wird, diese genomische DNA mit einer Restriktionsendonuklease gespalten wird,
die entstehenden Enden durch eine Ligationsreaktion mit bekannten, kurzen und doppelsträngigen, auch Adaptoren genannten DNA-Sequenzen versehen werden
Oligonukleotide, die gegen Bisulphit-behandelte Adaptoren komplementär sind, dazu verwendet werden, alle auf diese Art entstandenen DNA-Fragmente oder Sub-Populationen aus der Gesamtheit aller auf diese Art entstandenen Fragmente nach einer Behandlung mit Bisulphit, zu amplifizieren.

5. Verfahren nach Anspruch 1, **dadurch gekennzeichnet, daß** die Reaktion einer genomischen DNA-Probe mit einer Bisulphit-Lösung zwecks Umwandlung von Cytosinen zu Uracilen bei gleichzeitiger Erhaltung von Methylcytosin unter zyklischer Variation der Reaktionstemperatur bei Temperaturen zwischen 0 °C und 100 °C stattfindet.

6. Verfahren nach Anspruch 1, **dadurch gekennzeichnet, daß** die DNA-Probe vor der Behandlung mit Bisulphit in eine temperierbare, nur für kleine Moleküle durchlässige poröse Kapillare, in welcher die folgenden Reaktionsschritte der Bisulphitbehandlung durch Zu- und Abfuhr der Reagenzien durch Dialyse ausgeführt werden können.

7. Verfahren nach Anspruch 1, **dadurch gekennzeichnet, daß** die DNA-Probe vor der Behandlung mit Bisulphit in eine temperierbare, für kleine Moleküle undurchlässige Kapillare überführt wird, in welcher die folgenden Reaktionsschritte der Bisulphitbehandlung durch Zuund Abfuhr der Reagenzien durch Zuleitung von Reagenzien durch angeschlossene Kapillare ausgeführt werden können.

8. Verfahren nach Anspruch 1, **dadurch gekennzeichnet, daß** die sich an die Bisulphitbehandlung anschließenden Polymerase-Reaktionen in derselben Kapillare wie die Bisulphitbehandlung oder einer sich an diese Kapillare anschließenden Kapillare oder einem an diese Kapillare angeschlossenen Behälter durchgeführt werden.

9. Verfahren nach Anspruch 1, **dadurch gekennzeichnet, daß** in einer Kapillare, in welcher Polymerase-Reaktionen mit einer mit Bisulphit-behandelten DNA-Probe erfolgt auch eine Längentrennung der entstehenden Fragmentpopulation durchführt wird.

10. Verfahren nach Anspruch 1, **dadurch gekennzeichnet, daß** eine behandelte DNA durch Fällung des Bisulphit von diesem getrennt wird.

11. Verfahren nach Anspruch 1, **dadurch gekennzeichnet, daß** für die Amplifikation der Bisulphit-behandelten genomischen Proben-DNA Oligonukleotide zweier Klassen kombiniert werden, wobei die Oligonukleotide der einen Klasse die Base Cytosin oder deren Analoge nicht, ausschließlich im Kontext 5'-CpG-3', in nur sehr geringem, die Amplifikation nicht beeinflussendem Maße oder nur in die Amplifikation nicht beeinflussenden Bereichen der Oligonukleotide enthalten und wobei die Oligonukleotide der anderen Klasse die Base Guanin oder deren Analoga nicht, ausschließlich im Kontext 5'-CpG-3', in nur sehr geringem, die Amplifikation nicht beeinflussendem Maße oder nur in die Amplifikation nicht beeinflussenden Bereichen, wie zum Beispiel 5'-Bereichen der Oligonukleotide enthalten und wobei beide Klassen von Oligonukleotiden entweder
a) so kurz sind, daß in einer Amplifikation mit nur je einem Vertreter beider Klassen mehr als hundert verschiedene Fragmente amplifiziert werden oder
b) diese Oligonukleotide so viele degenerierte Positionen enthalten, daß in einer Amplifikation mit nur je einem Vertreter beider Klassen mehr als hundert verschiedene Fragmente amplifiziert werden oder
c) so viele Vertreter beider Klassen von Oligonukleotiden in einer Amplifikation verwendet werden, daß mehr als hundert verschiedene Fragmente amplifiziert werden.

12. Verfahren nach Anspruch 4 oder 11, **dadurch gekennzeichnet, daß** die behandelte und amplifizierte DNA in separaten Ansätzen zum Zwecke einer Polymerase-Reaktion mit in jeder Reaktion unterschiedlichen Oligonukleotiden gemischt wird, welche
an deren 5'-Termini zu den Adaptoren oder generell für die Amplifikation der Bisulphit-behandelten Oligonukleotide komplementär sind und
welche an deren 3'-Termini in jeder Reaktion unterschiedlich sind und
deren variable 3'-Termini hinter der bekannten Adaptor-Sequenz oder Oligonukleotid-Sequenz beginnen
und deren variable 3'-Termini über die bekannte Adaptor-Sequenz zwischen zwei und zwölf Nukleotide in die unbekannte Templat-DNA Sequenz hineinreichen.

13. Verfahren nach Anspruch 12, **dadurch gekennzeichnet, daß** solche Reaktionen, in welchen eine Polymerasereaktion mit Oligonukleotiden gestartet wird, welche zu einer mit Bisulphit behandelten DNA komplementär sind, außer den drei Nukleotiden dATP, dTTP und dCTP oder Analogen dieser drei Nukleotide
ein zur Base Cytosin komplementäres Nukleotid-Analog enthalten, welches nach Einbau durch die Polymerase jede weitere Strang-Verlängerung blockiert oder
gar kein zur Base Cytosin komplementäres Nukleotid oder Nukleotid-Analog enthalten.

14. Verfahren nach Anspruch 12, **dadurch gekennzeichnet, daß** solche Reaktionen, in welchen eine Polymerasereaktion mit Oligonukleotiden gestartet wird, welche zu einer mit Bisulphit-behandelten DNA komplementären DNA komplementär sind außer den drei Nukleotiden dATP, dTTP und dGTP oder Analogen dieser drei Nukleotide
ein zur Base Guanin komplementäres Nukleotid-Analog enthalten, welches nach Einbau durch die Polymerase jede weitere Strang-Verlängerung blockiert oder
gar kein zur Base Guanin komplementäres Nukleotid oder Nukleotid-Analog enthalten.

15. Verfahren nach Anspruch 12, **dadurch gekennzeichnet, daß** die Termination einer Polymerase-Reaktion an den Stellen, an denen vormals Methylcytosin in der DNA-Probe enthalten war, durch modifizierte Terminatoren vonstatten geht, und die Detektion der spezifisch terminierten Polymerase-Reaktionsprodukte über die Detektion der Modifikation erfolgt.

16. Verfahren nach Anspruch 1, **dadurch gekennzeichnet, daß** die verschiedenen, aus einer geeigneten Kombination der Ansprüche 3 bis 15 resultierenden Fragmentgemische der einzelnen Reaktionsansätze auf individuelle Punkte der Ionenquelle eines MALD1-TOF oder anderen Massenspektrometers aufgetragen werden und die Fragment-Zusammensetzungen der einzelnen Reaktionen durch Massebestimmung aller DNA-Fragmente bestimmt werden.

17. Verfahren nach Anspruch 1, **dadurch gekennzeichnet, daß** die verschiedenen, aus einer geeigneten Kombination der Ansprüche 3 bis 15 resultierenden Fragmentgemische der einzelnen Reaktionsansätze auf individuelle Bahnen einer Gelelektrophorese aufgetragen werden und die Fragment-Zusammensetzungen der einzelnen Reaktionen durch Längenmessung aller DNA-Fragmente bestimmt werden.

18. Verfahren nach Anspruch 4 oder 11, **dadurch gekennzeichnet, daß** die in Anspruch 12 definierten Oligonukleotide, mit denen Polymerase-Reaktionen gestartet werden, mit je Oligonukleotid unterschiedlicher Sequenz unterschiedlichen chemischen Markierungen gekoppelt sind und die Detektion und Unterscheidung der Polymerase-Reaktionsprodukte durch Analyse der chemischen und/oder physikalischen Eigenschaften der verschiedenen Markierungen mit gängigen chromatographischen oder massenspektrometrischen Verfahren erfolgt.

19. Verfahren nach Anspruch 18, **dadurch gekennzeichnet, daß**
die im ersten Amplifikationsschritt hergestellte Fragment-Fraktion der zu untersuchenden, Bisulphit-behandelten DNA mit zwei oder mehr chemisch unterschiedlich markierten Oligonukleotiden gleichzeitig gemischt wird,
diese Oligonukleotide in einem Reaktionsansatz als Primer für eine Polymerasereaktion benutzt werden,
die entstehende komplexe Mischung von Fragmenten in einem ersten analytischen Schritt einer elektrophoretischen Längentrennung unterzogen wird und
die einzelnen Längenfraktionen der aus der aus der Elektrophorese resultierenden Fragmentgemische einer chromatographischen oder massenspektrometrischen Analyse unterzogen werden, welche in jeder Längenfraktion die Präsenz oder Absenz der die Oligonukleotide charakterisierenden chemischen Markierungen detektiert.

20. Verfahren nach Anspruch 1, **dadurch gekennzeichnet. daß** auf eine Oberfläche Oligonukleotide aufgebracht werden, welche
entweder die Base Cytosin oder deren Analoge nicht, nur im Kontext 5'-CpG-3' oder nur in die Hybridisierung mit einer Proben-DNA nicht beeinflussenden Bereichen enthalten
oder die Base Guanin nicht, nur im Kontext 5'-CpG-3' oder in die Hybridisierung mit einer Proben-DNA nicht beeinflussenden Bereichen enthalten.

21. Verfahren nach Anspruch 20, **dadurch gekennzeichnet, daß** die Bisulphit-behandelte und nach entweder Anspruch 4 oder Anspruch 11 amplifizierte Proben-DNA
mit auf einer Oberfläche fixierten Oligonukleotiden hybridisiert wird, welche in an sich bekannter Art und Weise so auf dieser Oberfläche fixiert sind, daß an jedem Punkt der Oberfläche bekannt ist, welche Oligonukleotid-Sequenz sich an genau diesem Punkt befindet, - eine Hybridisierung der amplifizierten Proben-DNA mit den fixierten Oligonukleotiden nur dann erfolgt oder nach geeigneten Waschschritten bestehen bleibt, wenn Oligonukleotide und Proben-DNA in den für eine Hybridisierung wesentlichen Bereichen vollständig komplementär sind.

## Claims

1. Method for the characterization, classification and differentiation of tissues and cell types, for the prediction of the behavior of tissues and groups of cells, and for the identification of genes with modified expression, **characterized in that**
in genomic DNA, which has been obtained from any tissue sample, and which may have been treated, subjected to shearing, or cleaved by means of a restriction endonuclease in a manner which in itself is known, the base cytosine, but not 5-methylcytosine, is converted by treatment with a bisulfite solution into uracil, in a manner which in itself is known,
fractions of the so-treated genomic DNA are amplified by the use of nonspecific hybridizing oligonucleotides and/or a mixture of two or more different, specific hybridizing oligonucleotides and/or such oligonucleotides which are complementary to adaptor oligonucleotides that have been ligated to the end of the cleaved DNA before the bisulfite treatment,
overall, the quantity of the remaining cytosine on the guanine-rich DNA strand and/or guanines on the cytosine-rich DNA strand from the amplified fractions are detected by a hybridization or polymerase reaction, which is such that the data generated in such an analysis and automatically applied to a processing algorithm make it possible to draw conclusions regarding the phenotype of the analyzed cell material.

2. Method according to Claim 1, **characterized in that**
the data obtained from this analysis of several or many such tests on DNA samples from phenotypically identical or similar cells or tissue are correlated in a training phase using a neural network or other evaluation algorithm with the phenotype of the cells, whose DNA was examined,
the data included in this training phase in the evaluation pattern on the connection between the phenotype and the methylation state are used for deriving, by the generation of a methylation state of a DNA sample of unknown origin, the phenotype of the cells whose DNA was examined, or
the data included in this training phase in the evaluation pattern on the methylation state of the DNA of a known cell type are used for identifying cytosine positions which differ in the examined DNA from the methylation state determined in the training phase.

3. Method according to Claim 1, **characterized in that** the DNA is cleaved prior to the treatment with bisulfite and restriction endonucleases that contain cytosine in the 5'-CpG-3' context in their recognition sequence, and **in that** the DNA is cleaved only at those recognition sequences, in which cytosine, in the 5'-CpG-3' context, is in the unmethylated form in the 5' position.

4. Method according to Claim 1, **characterized in that**,
before the genomic DNA is modified in a manner which in itself is known with a bisulfite solution, this genomic DNA is cleaved with a restriction endonuclease,
the resulting ends are provided, by means of a ligation reaction, with known, short and doublestranded DNA sequences, also called adaptors,
oligonucleotides, which are complementary to the adaptors that have been treated with bisulfite, are used for the purpose of amplifying all the DNA fragments or subpopulations so generated from the totality of all the fragments produced in this manner after a treatment with bisulfite.

5. Method according to Claim 1, **characterized in that** the reaction of a genomic DNA sample with a bisulfite solution, for the purpose of converting cytosines to uracils while simultaneously obtaining methylcytosine, takes place under cyclic variation of the reaction temperature between 0 °C and 100 °C.

6. Method according to Claim 1, **characterized in that** the DNA sample, before the treatment with bisulfite, is transferred into a heatable porous capillary, which is only permeable to small molecules, in which the following reaction steps of the bisulfite treatment can be carried out by adding and removing reagents by dialysis.

7. Method according to Claim 1, **characterized in that** the DNA sample, before the treatment with bisulfite, is transferred into a heatable capillary that is impermeable to small molecules, in which the following reaction steps of the bisulfite treatment can be carried out by the addition and removal of the reagents by supplying reagents through connected capillaries.

8. Method according to Claim 1, **characterized in that** the polymerase reactions which follow the bisulfite treatment are carried out in the same capillary as the bisulfite treatment, or in a capillary connected to this capillary, or in a container connected to this capillary.

9. Method according to Claim 1, **characterized in that**, in a capillary in which the polymerase reactions are carried out with a DNA sample treated with bisulfite, a separation by length of the fragment population produced is also carried out.

10. Method according to Claim 1, **characterized in that** a treated DNA is separated by precipitation of the bisulfite from the latter.

11. Method according to Claim 1, **characterized in that**, for the amplification of the genomic DNA samples treated with bisulfite, oligonucleotides of two classes are combined, where the oligonucleotides of one class do not contain the base cytosine or analogs thereof, except in the 5'-CpG-3' context, or to only a very small degree, or only in regions of the oligonucleotides that are not essential for the amplification, and where the oligonucleotides of the other class do not contain the base guanine or analogs thereof, except in the 5'-CpG-3' context, or to only a very small degree, not influencing the amplification, or only regions, such as, for example, the 5' regions, of the oligonucleotides which are not influencing amplification, and where the two classes of oligonucleotides either
a) are so short that, in an amplification where each contains only one representative of the two classes, more than 100 different fragments are amplified, or
b) contain so many so-called degenerated positions that in an amplification with only one representative of each of the two classes, more than 100 different fragments are amplified, or
c) are used in such great numbers that, in an amplification, more than 100 different fragments are amplified.

12. Method according to Claim 4 or 11, **characterized in that** the treated and amplified DNA are mixed in separate preparations for the purpose of polymerase reactions, with different oligonucleotides in each reaction which
are complementary at their 5' termini to the adaptors or generally complementary for the amplification of the oligonucleotides treated with bisulfite, and which are different at their 3' termini in each reaction, and
whose variable 3' termini start downstream of the known adaptor sequence or oligonucleotide sequence,
and their variable 3' termini extend beyond the known adaptor sequence by 2-12 nucleotides into the unknown template DNA sequence.

13. Method according to Claim 12, **characterized in that** such reactions, in which a polymerase reaction with oligonucleotides is started, which oligonucleotides are complementary to DNA treated with bisulfite, contain, in addition to the three nucleotides dATP, dTTP and dCTP, or analogs of these three nucleotides,
a nucleotide analog that is complementary to the base cytosine and which, after incorporation by the polymerase, blocks any further elongation of the strand, or
no nucleotide or nucleotide analog that is complementary to the base cytosine.

14. Method according to Claim 12, **characterized in that** such reactions, in which a polymerase reaction with oligonucleotides is started, which oligonucleotides are complementary to DNA treated with bisulfite, contain, in addition to the three nucleotides dATP, dTTP, and dGTP, or analogs thereof, three nucleotides,
a nucleotide analog that is complementary to the base guanine and which, after incorporation by the polymerase, blocks any further elongation of the strand, or
no nucleotide or nucleotide analog that is complementary to the base guanine.

15. Method according to Claim 12, **characterized in that** the termination of a polymerase reaction, at the positions which earlier contained methylcytosine in the DNA sample, takes place by such terminators, and that the detection of the specifically terminated polymerase reaction products takes place by detection of the modification.

16. Method according to Claim 1, **characterized in that** the different fragment mixtures of the individual reaction preparations, resulting from an appropriate combination of Claims 3-15, are applied to individual points of the ion source of a MALDI-TOF or another mass spectrometer, and the fragment composition of the individual reactions is determined by determining the weight of all the DNA fragments.

17. Method according to Claim 1, **characterized in that** the different fragment mixtures of the individual reaction preparations, resulting from an appropriate combination of Claims 3-15, are applied to individual lanes in gel electrophoresis, and the fragment compositions of the individual reactions are determined by determining the lengths of all the DNA fragments.

18. Method according to Claim 4 or 11, **characterized in that** the oligonucleotides defined in Claim 12, by means of which polymerase reactions are started, are each coupled with an oligonucleotide having a different sequence and different chemical labels, and that the detection and differntiation of the polymerase reaction products takes place by the analysis of the chemical and/or physical properties of the different-labels by standard chromatographic or mass spectrometric procedures.

19. Method according to Claim 18, **characterized in that**
the fragment fraction, prepared in the first amplification step, of the DNA to be examined, which has been treated with bisulfite, is mixed simultaneously with two or more chemically differently labeled oligonucleotides,
these oligonucleotides are used in a reaction preparation as primers for a polymerase reaction,
the resulting complex mixture of fragments is subjected in a first analytical step to an electrophoretic separation by length, and
the individual length fractions of the fragment mixtures resulting from the electrophoresis are subjected to a chromatographic or mass spectrometric analysis, which detects, in each length fraction, the presence or absence of the chemical labels that characterize the oligonucleotides.

20. Method according to Claim 1, **characterized in that**, onto a surface, oligonucleotides are applied which either do not contain the base cytosine or analogs thereof, or only in the 5'-CpG-3' context, or only in regions which are not influencing the hybridization with sample DNA,
or which do not contain the base guanine, or contain it only in the 5'-CpG-3' context, or in regions which are not essential for hybridization with sample DNA.

21. Method according to Claim 20, **characterized in that** the DNA sample, which has been treated with bisulfite and amplified according to Claim 4 or Claim 11,
is hybridized with oligonucleotides that are fixed to a surface, which oligonucleotides have been fixed in a known manner to this surface so that it is known, for each point of the surface, which oligonucleotide sequence is located precisely at that point, a hybridization of the amplified sample DNA with the fixed oligonucleotides occurs, or persists only after appropriate washing steps, if oligonucleotides and the sample DNA are completely complementary in the regions that are essential for a hybridization.

## Revendications

1. Procédé de caractérisation, de classification et de différenciation de tissus et de types de cellules pour la prévision du comportement de tissus et de groupes de cellules, et en vue de l'identification de gènes modifiés dans leur expression,
**caractérisé en ce que**
dans un ADN génomique non traité, cisaillé, ou coupé à l'aide d'une endonucléase de restriction produit à partir de n'importe quel échantillon de tissu, d'un art et d'une manière connus en soi, la base cytosine, mais non pas la 5-méthylcytosine, par traitement avec une solution de bisulfite est convertie en uracile,
des fractions de l'ADN génomique ainsi traitées sont amplifiées, par utilisation d'oligonucléotides hybrida d'une manière non spécifique et/ou d'un mélange à base de deux ou davantage d'oligonucléotides différents hybrida spécifiquement et/ou de ces oligonucléotides qui sont complémentaires aux oligonucléotides d'agent d'adaptation, ligaturés avant le traitement par le bisulfite, sur les extrémités de l'ADN coupé,
à partir des fractions amplifiées on détecte au total une quantité de cytosine qui demeure sur le brin d'ADN riche en guanine et/ou de guanine sur le brin d'ADN riche en cytosine par une hybridation ou par une réaction avec une polymérase, de sorte qu'on analyse les données générées dans une telle analyse et converties automatiquement en un algorithme de transformation et on conclut pour un phénotype du matériau cellulaire analysé.

2. Procédé selon la revendication 1,
**caractérisé en ce que**
les données produites à partir de ces analyses de plusieurs ou de beaucoup de ces essais, sur des échantillons d'ADN provenant de cellules ou de tissus identiques ou similaires d'un point de vue phénotypique, sont mises en corrélation dans une phase d'entraînement par l'intermédiaire d'un réseau neuronale ou par l'intermédiaire d'autres algorithmes d'évaluation, avec le phénotype des cellules dont l'ADN a été étudié,
les données recueillies lors de cette phase d'entraînement dans les algorithmes d'évaluation par l'intermédiaire de la relation entre phénotype et modèle de méthylation, sont utilisées pour déduire par génération d'un modèle de méthylation d'un échantillon d'ADN d'origine inconnue, le phénotype des cellules dont l'ADN a été étudié, ou
les données recueillies dans cette phase d'entraînement dans les algorithmes d'évaluation par l'intermédiaire du modèle de méthylation de l'ADN d'un type de cellule connu, sont utilisées pour identifier quelles sont les positions de la cytosine qui s'écartent dans l'ADN étudié, de l'état de méthylation déterminé dans la phase d'entraînement.

3. Procédé selon la revendication 1,
**caractérisé en ce que**
l'ADN avant le traitement avec le bisulfite est coupé par ces endonucléases de restriction qui contiennent la cytosine dans le contexte 5'-CpG-3' dans leur séquence de reconnaissance, et qui coupent l'ADN seulement dans les séquences de reconnaissance dans lesquelles la cytosine en contexte 5'-CpG-3' non méthylée sur la position 5'.

4. Procédé selon la revendication 1,
**caractérisé en ce qu'**
avant que l'ADN génomique soit modifié d'un art et d'une manière connus en soi, avec une solution de bisulfite, cet ADN génomique est coupé avec une endonucléase de restriction,
les extrémités formées par une réaction de ligature sont pourvues de séquences d'ADN connues, courtes et à double brin, appelés aussi adaptateurs,
les oligonucléotides qui sont complémentaires vis-à-vis des adaptateurs, traités par le bisulfite, sont utilisés pour amplifier tous les fragments d'ADN qui se forment de cette façon ou tous les sous-fragments provenant de la totalité de tous les fragments formés de cette façon, après un traitement avec le bisulfite.

5. Procédé selon la revendication 1,
**caractérisé en ce que**
la réaction d'un échantillon d'ADN génomique avec une solution de bisulfite, se produit aux fins de la conversion des cytosines en uraciles avec obtention en même temps de méthylcytosine tout en variant d'une manière cyclique la température de réaction, à des températures comprises entre 0°C et 100°C.

6. Procédé selon la revendication 1,
**caractérisé en ce que**
l'échantillon d'ADN avant le traitement par le bisulfite est transféré dans un capillaire poreux, qu'on peut tiédir, seulement perméable pour de petites molécules, dans lequel les étapes suivantes de réaction du traitement par le bisulfite par amenée et évacuation des réactifs, peuvent être exécutées par dialyse.

7. Procédé selon la revendication 1,
**caractérisé en ce que**
l'échantillon d'ADN avant le traitement par du bisulfite est transféré dans un capillaire tiédissable, imperméable aux petites molécules, dans lequel les étapes de réaction suivantes par amenée et évacuation des réactifs de traitement par le bisulfite, peuvent être effectuées par admission des réactifs par des capillaires.

8. Procédé selon la revendication 1,
**caractérisé en ce que**
les réactions par la polymérase qui sont associées au traitement par le bisulfite, sont effectuées dans le même capillaire que le traitement par le bisulfite ou dans un capillaire qui est branché à ce capillaire, ou dans un récipient branché à ce capillaire.

9. Procédé selon la revendication 1,
**caractérisé en ce que**
dans un capillaire dans lequel les réactions avec la polymérase s'effectue avec un échantillon d'ADN traité par le bisulfite, on effectue aussi une séparation des longueurs de la population de fragments qui s'est formée.

10. Procédé selon la revendication 1,
**caractérisé en ce qu'**
un ADN traité est séparé du bisulfite par précipitation de celui-ci.

11. Procédé selon la revendication 1,
**caractérisé en ce que**
pour l'amplification des échantillons d'ADN génomique traités par le bisulfite, des oligonucléotides de deux classes sont combinés, dans lesquelles les oligonucléotides d'une classe ne contiennent pas la base cytosine ou ses analogues, sauf dans le contexte 5'-CpG-3' dans une mesure très faible qui n'exerce pas d'influence sur l'amplification ou seulement dans les zones qui n'exercent pas d'influence sur l'amplification des oligonucléotides, et dans lesquelles les oligonicléotides de l'autre classe ne contiennent pas la base Guanine ou ses analogues, sauf dans le contexte 5'-CpG-3' dans une proportion qui n'exerce pas d'influence sur l'amplification, seulement très faible ou seulement dans les zones qui n'exercent pas d'influence dans l'amplification comme par exemple les zones en 5' des oligonucléotides, et dans lesquelles les deux classes d'oligonucléotides soit :
a) sont si courtes que dans une amplification avec seulement un représentant des deux classes, plus de cent fragments différents sont amplifiés, soit
b) ces oligonucléotides renferment tant de positions dégénérées que dans une amplification avec seulement un représentant des deux classes plus de cent fragments différents sont amplifiés, soit
c) tant de représentants des deux classes d'oligonucléotides sont utilisés dans une amplification que plus de cent fragments différents sont amplifiés.

12. Procédé selon l'une des revendications 4 ou 11,
**caractérisé en ce que**
l'ADN traité et amplifié est mélangé dans des échantillons séparés aux fins d'une réaction avec la polymérase, à des oligonucléotides différents dans chaque réaction, qui
sont complémentaires sur leurs extrémités 5', aux adaptateurs ou en général pour l'amplification des oligonucléotides traités par le bisulfite et à leurs extrémités en 3' sont différents dans chaque réaction, et
dont les extrémités en 3' variables commencent derrière la séquence d'adaptateur connue ou la séquence d'oligonucléotide, et
dont les extrémités en 3' variables font pénétrer par l'intermédiaire de la séquence connue d'adaptateur entre deux et douze nucléotides dans la séquence d'ADN modèle, inconnue.

13. Procédé selon la revendication 12,
**caractérisé en ce que**
ces réactions dans lesquelles une réaction avec la polymérase est démarrée avec des oligonucléotides qui sont complémentaires à un ADN traité par le bisulfite en dehors des trois nucléotides, dATP, dTTP, et dCTP ou des analogues de ces trois nucléotides renferment un analogue de nucléotide complémentaire à la base cytosine lequel après insertion, est bloqué par la polymérase contre toute prolongation du brin, ou
ne contiennent même aucun nucléotide ou analogue de nucléotide complémentaire à la base cytosine.

14. Procédé selon la revendication 12,
**caractérisé en ce que**
ces réactions dans lesquelles une réaction par la polymérase avec des oligonucléotides est démarrée, qui sont complémentaires à un ADN analogue à un ADN traité par le bisulfite en dehors des trois nucléotides dATP, dTTP et dGTP ou des analogues de ces trois nucléotides, renferment un analogue de nucléotide complémentaire à la base guanine qui est bloqué après insertion, par la polymérase contre toute prolongation du brin, ou
ne contiennent même aucun nucléotide ou analogue de nucléotide, complémentaire à la base guanine.

15. Procédé selon la revendication 12,
**caractérisé en ce que**
la terminaison d'une réaction de la polymérase aux emplacements sur lesquels précédemment la méthylcytosine était contenue dans l'échantillon d'ADN procède grâce à des agents de terminaison modifiés, et la détection des produits de réaction de la polymérase terminés spécifiquement s'effectue par l'intermédiaire de la détection de la modification.

16. Procédé selon la revendication 1,
**caractérisé en ce que**
les différents mélanges de fragments résultant d'une combinaison appropriée des revendications 3 à 15 des échantillons de réaction individuels, sont appliqués sur des points individuels de la source d'ions d'un MALDI-TOF ou d'un autre spectromètre de masse, et les compositions de fragment des réactions individuelles sont déterminées par détermination de la masse de tous les fragments d'ADN.

17. Procédé selon la revendication 1,
**caractérisé en ce que**
les différents mélanges de fragments résultant d'une combinaison appropriée des revendications 3 à 15, des échantillons de réaction individuels, sont appliqués sur des travées individuelles d'une électrophorèse sur gel, et les compositions des fragments des réactions individuelles sont déterminées par mesure de la longueur de tous les fragments d'ADN.

18. Procédé selon la revendication 4 ou la revendication 11,
**caractérisé en ce que**
les oligonucléotides définis à la revendication 12, avec lesquels les réactions avec la polymérase sont démarrées, sont couplées avec un oligonucléotide de séquence différente ayant des marquages chimiques différents et **en ce que** la détection et la différenciation des produits de réaction avec la polymérase, s'effectue par l'analyse des propriétés chimiques et/ou physiques des différents marquages avec les procédés courants chromatographiques ou de spectrométrie de masse.

19. Procédé selon la revendication 18,
**caractérisé en ce que**
la fraction de fragment produit au premier stade d'amplification de l'ADN traité par le bisulfite à étudier, est mélangé en même temps à deux ou davantage, oligonucléotides marqués chimiquement d'une manière différente,
ces oligonucléotides sont utilisés dans un lot de réactions comme déclencheur pour une réaction avec la polymérase,
le mélange complexe de fragments qui se forme dans le premier stade analytique est soumis à une séparation de longueurs par électrophorèse, et les fractions de longueurs individuelles des mélanges de fragments résultant de l'électrophorèse sont soumises à une analyse par chromatographie ou par spectrométrie de masse qui détecte dans chaque fraction de longueur la présence ou l'absence des marquages chimiques qui caractérisent les oligonucléotides.

20. Procédé selon la revendication 1,
**caractérisé en ce qu'**
on applique sur une surface des nucléotides qui soit ne renferment pas la base cytosine ou ses analogues ou qui renferment seulement dans le contexte 5'-CpG-3' ou seulement avec des ADN d'essai dans l'hybridation avec des domaines qui n'exercent pas d'influence, ou
qui ne contiennent pas la base guanine, ou qui contiennent seulement dans le contexte 5'-CpG-3' ou dans l'hybridation avec des domaines qui n'exercent pas d'influence sur un ADN d'essai.

21. Procédé selon la revendication 20,
**caractérisé en ce que**
l'ADN d'essai traité par le bisulfite et amplifié selon soit la revendication 4 soit la revendication 11,
est hybridé avec des oligonucléotides fixés sur une surface, qui sont fixés d'un art et d'une manière connus en soi sur cette surface, de sorte qu'on connaît sur chaque point de la surface quelle est la séquence d'oligonucléotide qui se trouve exactement à ce point, une hybridation des ADN d'essai amplifiés avec les oligonucléotides fixés étant effectuée seulement ensuite ou laissée intacte après des étapes de lavage appropriées, si les oligonucléotides et les ADN d'essai sont totalement complémentaires dans les domaines essentiels pour une hybridation.
